# EUROPEAN PATENT APPLICATION

(11) **EP 3 865 849 A1**
(43) Date of publication of application: **18.08.2021**
(21) Application number: 20020073.1
(22) Date of filing: 14.02.2020
(51) Int. Cl.: G01N 15/14, B01L 3/00, C12N 5/071, G01N 21/84, G06K 9/00

(54) **SPERM PICKING SYSTEM**

(71) Applicant: Smart-Pick GmbH, 4051 Basel (CH)
(72) Inventor: KOSA, Gabor, 4051 Basel (CH)
(74) Representative: Pietruk, Claus Peter

(57) **Abstract**

A computer-assisted spermatozoon assessment method is suggested comprising the steps of feeding spermatozoon related digital data into a computerized spermatozoon assessment stage and automatically generating by the computerized spermatozoon assessment stage a spermatozoon assessment signal in response to the spermatozoon related data, wherein a sequence of digital spermatozoon images is fed into the computerized spermatozoon assessment stage as sperm related digital data and both spermatozoon motility and spermatozoon morphology are assessed by the computerized assessment stage in view of the digital spermatozoon images for generating the spermatozoon assessment signal.

## Description

The present application generally relates to the selection of particles, and in particular relates to a method and a device for sperm selection.

In human and animal reproduction medicine, techniques such as in vitro fertilization or intrauterine inseminations have become common. For such techniques, sperm of a high quality is needed, that is sperm, that has a high likelihood of fertilizing. However, in semen both spermatozoa having a high likelihood of fertilizing and spermatozoa having a low likelihood of fertilizing can typically be found, cmp. e.g. Cooper TG, Noonan E, von Eckardstein S, Auger J, Baker HW, Behre HM, et al. "World Health Organization reference values for human semen characteristics.e Hum Reprod Update. 2010; 16:231. According to the WHO regulation a sperm sample with 4% normal morphology or more is considered normal sperm, cmp. "World Health Organization. WHO Laboratory Manual for the Examination and Processing of Human Semen, 5th edn. Geneva, CH: World" Health Organization, 2010.

As the success rate of fertilization procedures increases with the quality of sperm, standards have been developed for evaluating sperm quality and methods have been developed for selecting sperm of a high quality.

Regarding sperm quality, reference is made to the "WHO laboratory manual for the Examination and processing of human semen" 5th edition. In the manual, it is stated that immediately after ejaculation, semen is typically a semisolid coagulated mass, but that within a few minutes at room temperature, the semen usually begins to liquefy; it is stated that a sample typically liquefies completely within 15 minutes and that liquefaction can be recognized both macroscopically and microscopically. It is suggested to visually assess semen viscosity after liquefaction.

It is also stated that a normal liquefied semen sample has a homogeneous, grey-opalescent appearance. Then, for assessment of semen quality, an initial microscopic investigation is suggested to provide an overview of the sample to reveal strand formation, sperm aggregation or agglutination, the presence of cells other than spermatozoa and, using a higher magnification, assessment of sperm motility and determination of the dilution required for accurate assessment of sperm number. Regarding the assessment of sperm motility, it is suggested to assess approximately 200 spermatozoa in a replicate using a phase contrast microscope; systematic errors that can occur in visually assessing sperm motility are discussed in the manual. Regarding sperm vitality, it is suggested that a staining technique is used, but other tests such as hypo-osmotic swelling are also suggested for the assessment of vitality, in particular when choosing spermatozoa for intracytoplasmic sperm injection where staining of spermatozoa must be avoided. In the hypo-osmotic swelling assessment, a semen sample is mixed with a swelling solution and then, swollen dead cells are optically distinguished from swollen vital cells. It is noted that different types of swelling can be observed, leading to different typical morphological changes in human spermatozoa. It is noted that for morphological analysis, it is customary to prepare semen smears that are air-dried before fixation and staining. As with motility, it is suggested to assess sperm morphology by also evaluating at least 200 spermatozoa in each replicate.

The WHO document notes that semen samples contain spermatozoa with different kinds of malformations and that defective spermatogenesis and some epididymal pathologies are commonly associated with an increased percentage of spermatozoa with abnormal shapes. Reference is also made to the "Atlas of Human Sperm Morphology Evaluation", Edited by Thinus F.Kruger MD, FRCOG and Daniel R.Franken PhD, Tygerberg, Republic of South Africa, Taylor & Francis
The document also notes that it is possible to measure sperm concentration by computer aided sperm analysis, stating that sperm motility and kinematics can be measured using computer aided sperm analysis. Reference is made to a computer aided sperm analysis instrument detecting and counting fluorescent sperm heads. In this context, the document alleges that without microscopic evaluation, there is no way of knowing if the spermatozoa are intact, noting that image analysis has the potential to bring about major advances in quantification, objectivity and reproducibility in the assessment of sperm morphology, stating that commercial systems would be available for quantifying the morphology of the sperm head and midpiece, and possibly the principal piece. However, it is stated that tail defects affecting motility could be more directly assessed by using CASA to measure motility and motion.

Regarding sperm preparation techniques, the selection of spermatozoa by their ability to swim out of seminal plasma and into culture medium is known as the swim-up technique. The WHO handbook suggests that direct swim-up techniques are often used when semen samples are considered to be largely normal, whereas density gradients may be preferred in other cases, because of the greater total number of motile spermatozoa recovered.

The handbook states that for quality assurance of sperm motility assessments, specimens video recorded on tape, CD or DVD can be used; it is suggested that video recordings should be of a magnification similar to that observed in the microscope when actual specimens are analyzed. It is also stated that for daily routine assessment the use of a television camera and screen at the same magnification increases the validity of the recordings for quality control.

Note that the document lists a number of parameters for assessing sperm quality. While there are a number of parameters considered relevant, several of these do not relate to single sperm cells assessment, but relate to the overall ejaculate sample, for example the time for liquefaction, the viscosity of the ejaculate, the volume thereof, the color, the pH and the sperm concentration; the same holds for the lykocyte concentration found in the ejaculate. However, WHO also suggests to assess motility, vitality and morphology, which requires assessment of the respective parameters for single sperms.

US 6,833,542 B2 relates to a method for sorting particles. Reference is made to biological particles, mentioning inter alia sperm cells and cancer cells. Mobile sperm cells are explicitly mentioned, stating that one of the reasons for male infertility is the lack of high enough percentages of viable and/or mobile sperm cells. The document alleges that viable and/or mobile sperm cells can be selected and that by enriching them, higher rates of fertilization could be achieved. The document intends to simultaneously analyze and isolate specific cells based on optophoretic parameters. It is suggested to electrokinetically move particles and subject the particles to optical forces for sorting; the electrokinetic forces mentioned include eletroosmosis, electrophoresis and dielectrophoresis.

In one example, red and white blood cells are sorted in micro-channels; an example is a given where particles from different materials are diluted in distilled water, images thereof are captured using a CCD camera and image software is used to verify that particles of one material had moved. It is also suggested that an imaging system includes a CCD detector as well as image enhancement and image analysis software and that a control system controlling the generation of a force pattern for particle isolation is coupled thereto, creating a feedback system to control the operation of the overall system.

US 7,402,131 B2 generally relates to techniques and systems for separation of particulate or cellular materials such as blood or semen and suggests a multiple laminar flow-based particle and cellular separation method with laser steering and using optical traps. It is suggested that the movement of particles may be monitored via an optical data-stream and that the optical data-stream may be used by a computer program that controls the operation of optical traps.

From WO 2004/0882838 A2, a system for analyzing particles using multiple flow cytometry units is known which is stated to yield sperm populations that are enriched with sperm cells having desired characteristics, such as viable populations of sperm cells sorted according to DNA characteristics for use by the animal production industry to preselect the sex of animal offspring. It is noted in the document that to preselect the sex of animal offspring, flow cytometry can be used to sort X and Y sperm cells, but that a commercially successful high throughput system is difficult to achieve. The document suggests flow cytometry to provide information for classifying particles passing in single file in a fluid stream through the measuring device and suggests that the particles may be separated into populations using techniques such as droplet sorting, droplet interference sorting and fluid switching and to selectively destroy unwanted particles for example by fertilization. It is suggested that in an optically based flow cytometry system optics are used to direct and focus beams of light on the screen containing the particles and collect emissions from the particles such as scattered light or fluorescence emissions. The authors note that such systems are expensive. In view of this, the known system includes a plurality flow cytometry unit operable to classify particles in a stream of fluid using a beam of electromagnetic radiation and a sensor operable to generate a time-varying output signal indicative of at least one characteristic of the particles in the stream of fluid.

US 9,757,7268B2 relates to a system for high throughput sperm sorting in a microfluidic chip wherein sperm is aligned and oriented in the flow channel, sperm orientation is determined and relative DNA content is measured for analysis and/or sorting. In the introductory portion, it is stated that in the livestock production industry, a gender pre-selection provides an economic benefit, but that the number of sperm cells that can be sorted for X-chromosome or Y-chromosome bearing sperm is limited in practice. It is stated that in bovine, conventional artificial insemination doses may contain about 10 Mio sperm cells whereas sex-sorted doses often contain about only 2 Mio sperm cells; it is stated that e.g. for porcine artificial insemination, the doses are in the magnitude of hundreds of millions and billions of spermatozoa respectively. It is then noted that sorting is time sensitive and may injure sperm, for example due to the high velocities in fluid streams in which sperm travel. This is stated to give rise to damaging shearing forces.

It is then suggested that electromagnetic radiation from sperm aligned and oriented in a microfluidic channel is captured by collection optics and projected onto a detector for quantification by an analyzer. It is also suggested in this context to cool sperm for the purpose of reducing sperm activity which may misalign and unoriented sperm. The prior art document notes that while some shade variation exists from species to species, a general shape is given for mammalian sperm. It is stated that the 2 largest portions of the sperm cell are the sperm tail and the sperm head housing the nuclear DNA to which selective dyes for sex-sorting sperm bind advantageously. The document notes that differentiating sperm nonetheless is difficult in many species because the uptake of DNA selective dye differs only slightly between X-chromosome bearing sperm and Y-chromosome bearing sperm, and that if sperm become unaligned or unoriented, their measured fluorescence fluctuates significantly. Regarding the measurement of sperm properties, the document emphasizes that sperm are living, motile cells which may be erratically propelled by motion from their tail. The authors note that using multiple flow channels in a single chip do not allow to illuminate the sperm head from all sides or head-on and discuss the form of a fluorescent signal pulse.

In US 10,105,712 B2 methods and devices for sorting cells and other biological particulates are described. In the introductory portion of the document, it is stated that the efficient separation of biological particulates such as stem cells, embryos or bacteria from either relates without damaging the particulates is an important step in a variety of diagnostic and treatment methods. It is stated that the sorting of non-motile viable sperms from non-motile non-viable sperm may be useful in a subset of infertile human patients or animals, when nearly all sperm are non-motile. It is then stated that for sorting, the motion of motile particles in an external nonuniform electric field (dielectrophoresis) does not require that the cell itself be motile.

The document notes that the dielectric potential of any cell has been shown to depend e.g. on its physiological status, composition, cytoplasm contents, organelles and phenotype, in addition to the frequency of an electrical field applied. The document alleges that there is a need for facilitating sorting of heterogeneous populations of living and dead cells as well as selecting by viability, cell size, magnitude of cell membrane dipole and features of the cells chromosome content such as chromosome number, degree of chromosome damage, chromosome type (gamete sex sorting], and genetic aberrations of known and unknown diseases. In order to create inhomogeneous electric fields, a microfluidic device with photoconductive electrodes onto which suitable light patterns are projected is used and it is suggested that the position and timing of light patterns can be determined dynamically in response to feedback received from registering the position and dielectrophoresis response of particles; the document notes that the particle position may be registered using microscopic imaging and that a recorder for collecting data from the microscopic imager may be provided with a personal computer having software to control the light pattern and other parameters such as a microfluidic pump. It is explicitly stated that the technology can be used for sperm sorting.

US 2012/0301869 A1 relates to particle separation devices, methods and systems. In introductory portion, it is stated that relatively small differences in sperm DNA content are difficult to distinguish, leaving a significant portion of a sperm sample unidentified as either X-or Y-bearing sperm, so that where a high purity of sperm is required, fewer sperm are sorted which is stated to be unacceptable for sperm in limited supply. A flow cytometer is suggested wherein a video camera could be used to enable an operator to monitor particles sorting and to see what types of adjustment need to be made; however, it is also stated that he characteristic of X-chromosome bearing sperm is that they tend to absorb more fluorochromes dye then Y-chromosome bearing sperm and as such, the amount of light emitted by laser-excited absorbed dye in the X-chromosome bearing sperm differs from that of the Y-chromosome bearing sperm.

US 2014/0099664 A1 relates to a high-efficiency method of sex sorting sperm, using e.g. a jet-in-air flow cytometer. When discussing staining of sperm, it is suggested that at the time of collection, thawing or even pooling, sperm may be checked for concentration, pH, motility and/or morphology. It is then suggested that separation can be effected in view of detected fluorescence.

In US 2015/0079676 A1, a method and apparatus for the isolation of motile sperm is described. It is suggested to deliver a fluid containing sperm into a micro-volume at least partially defined by a wall which includes a wall termination or change in angle away from the micro volume and to then allow at least some motile sperm to move along side wall and to exit the micro-volume by changing direction away from the micro volume.

From US 2016/0290913 A1, a system and method for sperm sorting is known. It is stated that assisted reproductive technology requires selection of healthy sperm that is not only highly motile, but has also normal morphology, mature nuclei and lesser reactive oxygen species production. It is noted that IVF/ICSI procedures can be compromised if the sperm selected are abnormal and that microfluidic sperm sorting devices having very low throughput can only process small semen volumes such as 2 µL to 50 µL, limiting the application to reproductive clinics where sperm samples can have volume of 1,5 ml. It is then stated that the vaginal mucus forms tiny micro channels that help guide sperm through to the egg and that exhaustion as a mechanism for sorting sperm has been experimentally and theoretically demonstrated to leverage exhaustion to sort healthy sperm. It is suggested that a microfluidic system includes a filter with micropores arranged in the flow paths between the inlet and the outlet to cause sperm traveling along the flow paths to move against through the filter and gravity to reach the outlet. It is then stated that sperm can be simultaneously sorted, monitored and evaluated using a wide field of view, lensless imaging technology utilizing shadow imaging with an imaging sensor such as a CCD or CMOS, allegedly allowing detecting/counting and monitoring in real-time the dynamic location of hundreds of thousands of individual cells over an ultrawide field of view. It is specifically noted that the standard microscope would be unable to monitor a whole microfluidic sorting chip and analyze sperm and real-time. According to the document, the quality of separated sperm is evaluated following the separation.

From US 2017/0205390A1, an interferometric system and method for use with biological cells and organisms including sperm is known. It is suggested to determine a topographic optical phase delay map of a label free sperm, determine at least one physical parameters of the label free sperm and generate data indicative of sperm quality for label free sperm. It is stated that after an analysis stage, selection is done on each cell individually in a cell-by-cell fashion. The document mentions physical parameters such as a total volume of the cell had, the width and length of the head area, the centroid and waited centroid of each had region, 2-dimensional shape features such as form factor, roundness, aspect ratio, effective diameter, roughness and so forth.

From US 2018/0 266937 A1, for selection of semen a microfluidic device is known, wherein for sensing a characteristic of a sperm cell, an analyzing zone is provided comprising both electrodes and an optical lens, so that in a sorting zone, a sperm cell can be directed towards one of several outlets based on an analysis. Acquired video and impedance data are timestamped for matching. For sperm tracking image analysis, use of the "motion-based multiple object tracking function" of a computer vision system toolbox in Matlab is suggested, whereas cell orientation and morphology are determined by impedance analysis,

From 2019/0024045 A1, a micro-fluidic device for sorting of highly motile and morphologically normal sperm from unprocessed semen is known. The microfluidic chip is suggested to be provided with one or more inlet chambers and sperm collection outlet chambers, and a channel having various micro-fabricated structures in the middle in different geometrical shapes and orientations and with varying periodicities and patterns. According to the document, structures periodically placed inside a channel change the ways the sperm move, with sperm having a deformed morphology interacting and following pathways different from those of sperm with normal morphology. Also, it is stated that sperm cells are observed to move fast in comparison to their counterparts in the inlet or outlet in the pillar geometry due to effects including hydrodynamic interactions. The fluidic channel described is without movement or flow and the fluid inside not actively driven.

While the above shows that in the prior art methods and devices have been suggested to allow selection of spermatozoa having a high likelihood of fertilizing, to this day, the success rate of artificial insemination and in vitro fertilization is still rather low. In part, this may be due to deficits in the selection process, indicating that improved methods and devices might be beneficial. Accordingly, there is still a need for an improved selection of spermatozoa, in particular a fast and/or a precise selection of spermatozoa. This holds particularly for in vitro fertilizations (IVF) such as an intracytoplasmic sperm injection (ICSI) process as these currently are used in particularly critical cases where other, more conventional methods such as artificial (intraperitoneal) insemination methods have failed before.

It is an object of the present invention to provide novelties for the industrial application.

This object is achieved by the independent claims. Some of the preferred embodiments are described in the dependent claims.

In one embodiment, a computer-assisted spermatozoon assessment method is suggested comprising the steps of feeding spermatozoon related digital data into a computerized spermatozoon assessment stage and automatically generating by the computerized spermatozoon assessment stage a spermatozoon assessment signal in response to the spermatozoon related data, wherein a sequence of digital spermatozoon images is fed into the computerized spermatozoon assessment stage as sperm related digital data and both spermatozoon motility and spermatozoon morphology are assessed by the computerized assessment stage in view of the digital spermatozoon images for generating the spermatozoon assessment signal. Note that rather than referring to an assessment, reference could also be had to for example a classification or binning. Accordingly, instead of referring to assessing a sperm, reference could be had to classifying the sperm quality. Note that it is not necessary to diagnose the reason why spermatozoa in the sample might have a low quality.

There are a number of applications where such computer assisted spermatozoon assessment method is useful. First of all, the assessment signals of a plurality of spermatozoa could be stored so that a statistical evaluation of the entity of spermatozoa assessed can be effected. This might help to identify reasons why a person is infertile. It is also possible to quantify certain parameters such as the degree of infertility of a male patient. Then, for pure research, a data base could be established wherein spermatozoon morphological and motility data are gathered for a plurality of male persons. This is particularly useful in cases where the data can be correlated or in other ways combined with further data characteristic of persons whose spermatozoa have been assessed. Another important application is to help in the selection of spermatozoa suitable for fertilization. It should be kept in mind that according to WHO, compare Kumar, Naina, and Amit Kant Singh. "Trends of male factor infertility, an important cause of infertility: A review of literature", Journal of human reproductive sciences 8.4 (2015): 191, even for a healthy male human person, more than 96% of the spermatozoa have some defect that may lead to failing in fertilization attempts; it will be understood that the percentage is even higher, frequently significantly higher in persons that have some defects; if a fertilization is to be attempted using the spermatozoa of such donors, the success rate could be significantly increased using a suitable assessment of spermatozoa. This holds in particular for ICSI (intracytoplasmic sperm injection) fertilization methods where a single spermatozoon is first singled out and then used for intracytoplasmic sperm injection. It is noted that rather than identifying a single suitable spermatozoon, a small number of spermatozoa such as less than 5, 10,15, 20, 30, 50 or 100 spermatozoa could be selected, the small number being however larger than 1 spermatozoon, or larger than 5,10,20,30,40, 50 spermaozoa respectively. Given the vast number of spermatozoa typical for semen, identifying some suitable spermaotozoa such as less than 5, 10,15, 20, 30, 50 less than 100 or less than 1000 spermatozoa can be considered singling out spermatozoa.

It should be noted that the computer assisted assessment suggested here need not be combined with a specific sorting arrangement such as a specific microfluidic device; it is feasible to record images with a camera essentially covering completely the field of microscopic view a laboratory technician has who is selecting suitable spermatozoa for an ICSI fertilization basically by hand. In such a case, the camera images could be assessed in a suitable assessment stage for assessing one or even a plurality of spermatozoa simultaneously, and the method could be used to virtually enhance a real image the laboratory technician is observing. For example, an image could be displayed to the laboratory technician wherein a plurality of spermatozoa currently close to a pipette used to capture a healthy spermatozoon are colored depending on their assessment, e.g. in red color for spermatozoa that have some defect and should not be used, in green color for spermatozoa that have been found to have a high quality and in yellow for spermatozoa that currently have not been assessed, for example because they are not in focus, have just entered the field of view, are too close to other spermatozoa to allow an assessment or because track has been lost.

In a case where only the assessment is needed, but no sorting is to be effected automatically, it is possible to either assess the sequence of images close to the place where the images are acquired; for example, a laboratory microscope used for an ICSI process and having an electronic display could be provided with a suitable computer assessment stage executing suitable assessment steps; on the other hand, it would also be possible to transfer the sequence of images via a sufficiently broadband connection to an assessment center spaced apart from the laboratory and to assess the sequence of images in this assessment center, giving real-time feedback to the laboratory technician, for example by supplying a video stream wherein spermatozoa suitable for infertilization are marked. Also, it would be possible to assess spermatozoa in a non-real-time manner where the emphasis is on identifying morphological and/or motility-related deficiencies of a spermatozoa sample, for example in order to provide a medical doctor with information that allows to identify a reason for infertility. Note in this respect that it is not necessary that the assessment signal is indicative for one of a plurality of specific reasons for infertility, although it is considered possible to establish for example a ranking of probabilities for specific reasons for infertility to be present in a given male person.

Although it is not necessary to automatically actually sort spermatozoa in response to the assessment, it will be understood that the assessment and the assessment signal generated in response to the assessment may well be used for automatic sorting. In more detail, it is possible to derive a sorting or selection signal; accordingly, an automatic decision can be made in view of the assessment signal whether to include a spermatozoon in a fertilization process or whether to exclude the spermatozoon from further use and a corresponding selection or sorting signal can be derived should the assessment signal itself be not usable as such, for example because sorting signal following another protocol, having another voltage level than the assessment signal or the like is needed.

In another embodiment, a computer-assisted spermatozoon selection method is suggested comprising the steps of transmitting spermatozoon related digital data for input into a computerized spermatozoon assessment stage and receiving a spermatozoon selection signal automatically generated by the computerized spermatozoon assessment stage in response to the spermatozoon related data, wherein a sequence of digital spermatozoon images are transmitted to be inputted into the computerized spermatozoon assessment stage as sperm related digital data and the selection signal received is indicative of an assessment of both spermatozoon motility and spermatozoon morphology in view of the digital image data. From what is stated above, it would be understood that it is not necessary to effect the assessment close to the place where the sequence of digital spermatozoa and images are acquired; in such a case, transmitting spermatozoon related digital data for input into the computerized spermatozoon assessment stage would use e.g. a long-distance communication line or a wide area network communication line, for example an or an internet connection such as a connection using a TCP/IP standard and having a sufficient bandwidth. Note that although bandwidth must be sufficient and latency must be sufficiently low, in particular where a near real-time assessment is needed, generally neither the frame rate nor the resolution has to be particularly high. Where the frame rate of a camera used with a laboratory microscope or other device is used for spermatozoa selection is higher, in particular significantly higher such as at least 2 times higher, 3 times higher, 4 times higher than needed for spermatozoon assessment, the sequence of images can be "thinned out" to save bandwidth; also, where the resolution is higher, in particular significantly higher such as at least twice as high, 3 times as high, 4 times as high as needed for spermatozoon assessment, it would be possible to transmit a sequence of images having a resolution lower than the resolution of the images acquired. It will be understood by a person skilled in the art that the frame rate needed to evaluate in particular motility of spermatozoa will depend inter alia on the magnification and on the overall field of view. The frame rate should be such that a plurality of images of one and the same spermatozoon is found in a sequence; where more than one or very few spermatozoa are simultaneously in the field of view, the frame rate and resolution also have to be sufficiently high so as to allow tracking of spermatozoa, at least in case where a user of the method wishes to select spermatozoa that have been in the field of view of the assessment.

It will be understood that in certain instances, the sequence of images can also be evaluated at the location where the digital image data are required. For example, a spermatozoon sorting system may comprise a microfluidic arrangement, a camera for acquiring images of spermatozoa containing fluid passing through the microfluidic arrangement with a sufficiently high resolution and frame rate as well as an assessment stage for assessing the sequence of digital images, and a selection stage for selecting spermatozoa in accordance with the assessment. Here, the transmission can be effected using a simple electrical connection, a data signal bus, a wireless connection to a wireless interface of the assessment stage and so forth.

With respect to the digital spermatozoon images (or the sequence of digital spermatozoon images), it will be understood that these images may have any digital format. It is possible for example to provide the sequence of digital images as a sequence of raw format still photographies, DNG format still full photographies, PNG format still full photographies, JPEG format still photographies, TIFF format still photographies, GIF images or the digital spermatozoon images could be provided as video streams such as MPEG encoded images. Note that the data could also be encrypted, in particular where the images are transmitted via a WAN network such as the internet. It will thus be understood that instead of making reference to the use of digital spermatozoon images, reference could also be had to data relating to digital spermatozoon images; however, it is believed that referring to spermatozoon images or digital spermatozoon images or a sequence of spermatozoon images or a sequence of digital spermatozoon images allows the invention to be understood more easily than referring to e.g. spermatozoa images data, digital spermatozoon images digital data, the digital sequence of spermatozoon images data and so forth.

In a preferred embodiment, a sequence of at least three, preferably at least four frames are fed into the computerized spermatozoon assessment stage. It will be understood that providing a sufficient number of frames for the sequence is advantageous with respect to the assessment of motility, given that the motility can be assessed based on a higher number of frames and thus can better describe movement of a spermatozoon; however, providing a sufficient number of frames for assessment of morphology also improves the results. While the spermatozoa move within the field of view, it is likely that they will be observed from different angles and/or from different sides; accordingly, observing them in a number of frames will allow better observation resulting in better characterization.

Where certain morphologic parameters such as length of the tail, the 3d shape of the head, and so forth can be established in several frames of the sequence, it is possible to establish a respective morphological parameter using statistical methods such as averaging, weighted averaging or rejection of outliers. Furthermore, using a sufficient number of frames (or images) in a sequence allows tracking of spermatozoa; this is of particular importance where a plurality of spermatozoa is simultaneously observed in the field of view and where in the images acquired constituting the sequence, more than a single spermatozoon can be found. In such a case, it must be ensured that observations relating to spermatozoa in one frame of the sequence and observations relating to spermatozoa in another frame of the sequence are assessed in a manner such that only observations relating to the same spermatozoon are combined; it will be understood by a person skilled in the art of image analysis that techniques for tracking exist, for example using motion image history. Where a plurality of spermatozoa are simultaneously present in the field of view, a plurality of possibilities exists with respect to the assessment. For example, in assisting a laboratory technician in selecting a single spermatozoon for ICSI fertilizations, by generating a stream of enhanced reality images where the enhancement consists of marking at least some spermatozoa assessed positively by the assessment stage, only those spermatozoa that have been tracked without any reasonable trace of doubt and have been assessed to be of sufficiently high quality need to be marked, while all other spermatozoa need not be marked; accordingly, even if track is lost for some of the spermatozoa within the field of view, sorting or selecting can still be effected.

In contrast, where an automatic sorting is to be effected based on the assessment, and where a plurality of spermatozoa are observed simultaneously within a field of view, for example because the dilution of the sample has not been sufficient, different decisions are possible with respect to whether or not a given currently assessed fraction of the sample is to be discarded or kept for future use and hence sent to for example a first or a second reservoir. For example, a decision whether or not to transfer a given currently assessed fraction of the sample to a first reservoir for future use or to another reservoir for discarding could take into account whether or not a sufficiently high number of spermatozoa thus far has been detected and/or whether the current assessment is a first or early assessment of a multipass assessment rather than a final or late stage pass. Accordingly, with respect to tracking, for directing an entire group of spermatozoa into a reservoir for future use it might be sufficient that a single (usable) spermatozoon can be identified and tracked at least to the degree that it is known that the selected single sperm still is part of the respective entire group; alternatively, it might be required that a majority of spermatozoa concurrently observed are of a high or low quality respectively to warrant future use or to warrant discarding the aliquot.

In a preferred embodiment, spermatozoa are sorted in response to the spermatozoon assessment signals. In particular, spermatozoa can be automatically sorted in response to spermatozoon assessment signals. The automatic sorting allows for a high throughput of samples. This in turn allows for repeating the assessment using a plurality of passes. This is advantageous as it allows to refine the selection more and more by using more assessment parameters and/or stricter boundaries, increasing the overall quality of the final fraction selected; as the initial sorting pass or early sorting passes can be carried out rather fast, using for example higher throughput/flow rates through a microfluidic device used in sorting and/or a lower number of frames in a sequence. It will be understood by a skilled person that reducing the number of frames in a sequence has advantages both as the computational effort needed for generating an assessment signal is lower, typically scaling with the number of frames in at least a linear manner, and that also reducing the number of frames reduces the time needed for framing acquisition and hence increases the throughput. It will also be understood by a skilled person that a plurality of passes can be executed and that some of these passes need not rely on the simultaneous assessment of both motility and morphology; rather, it could suffice if in one or a few passes only one of motility and morphology is respectively assessed for selection of spermatozoa, while at least one pass assesses both motility and morphology in response to images data acquired for that pass.

If in a preferred embodiment, at least one fraction of spermatozoa sorted in a first assessment pass is subjected to at least one further assessment (and sorting) pass, it is further preferred if during the further or a further assessment pass, at least one of spermatozoon motility and spermatozoon morphology are assessed in a manner different from the manner of assessment during the first pass, in particular such that during a later pass at least one morphology characteristic different from all previously assessed morphology characteristics is assessed and/or a threshold for evaluation of a morphology characteristic previously assessed is set more stringent than for a previous pass and/or wherein a more stringent threshold for minimum spermatozoon motility is set for spermatozoa to be selected. It will be understood that the throughput through sorting devices is high enough to allow for a plurality of passes such as 2, 3, 4 or 5 passes even in cases where the percentage of high quality spermatozoa is not particularly high. As the repeated passes can be fully automized, for example by flushing back fluid from a first reservoir in which higher quality spermatozoa have been selected, into the initial (emptied) reservoir of fluid containing spermatozoa to be assessed, by repeating the sorting, the repeated pass does not require a significantly longer laboratory technician time for execution. Another possibility exists to have a preselection stage on the microfluidic device and at least one further selection stage on the same microfluidic device, so that the first stage, some of the spermatozoa can be selected by transferring them into the at least one further selection stage; preferably, in both selection stages, the sequence of images is acquired and assessed both with respect to morphology and motility. Also, even though a plurality of microfluidic devices are needed and although for each sample analyzed, certain additional hardware is also required, such as an image sequence acquisition camera and fluid steering means adapted to be controlled in response to an assessment signal, the overall amount of hardware needed is typically acceptable in view of the higher success rate due to the higher quality quickly obtained in a multipass and/or multistage assessment.

It will be understood that in a preferred embodiment, the computer-assisted method will comprise the step of acquiring sequences of images of spermatozoa to be assessed. However, as has been indicated above, it would be possible that the acquisition is taking place spaced apart from the actual computer assisted assessment of the images; in certain cases, it would even be possible to acquire the images in one country, transmit the sequence of images via a broadband communication line to another country, assess the images in that other country and send back the results of the assessment. However, in an integrated system, the computer assisted assessment will usually take place at the same location where the images are taken, for example with in the same laboratory room the microfluidic device is placed or in a server room adjacent or close to the laboratory. It will typically be preferred if the acquisition of images can be controlled by a controlling means, for example a microcontroller also controlling the illumination of the field of view, in particular controlling a flash or strobe for illumination, as well as a sorting device and/or a device for causing the spermatozoa containing fluid to pass through the microfluidic arrangement. It will be understood that such control will profit from the smaller latencies typically associated with smaller distances between the devices controlled and the controlling circuits: for example, where a clear decision can be derived from an assessment of a sequence comprising only 3 or 4 images, it will not be necessary to acquire additional images for the sequence; however, in case a clear decision cannot be made in view of the first 3 or 4 images of the sequence, a method might be implemented in a manner such that 1, 2, 3 or more additional images are acquired and assessed, preferably assessed together with the initial 3 or 4 images. If this is done in an embodiment where the assessment is effected far away from the laboratory, the latencies introduced for data transmission might significantly prolong the analysis of a given sample. Accordingly, in such a case, it would be preferred to have the data processing units used for implementing the computer-assisted assessment close by. In a preferred case, the data processing unit or at least part of the data processing units, for example for a preprocessing determining whether certain characteristics such as roundness, length of tail and so forth can be determined with a sufficient precision from images acquired would be integrated into a sorting device or sorting arrangement placed in a laboratory.

It will be understood that in a sorting device, the means for causing spermatozoa-containing fluid to pass through the microfluidic arrangement may in particular be suction or pressure pumps. The spermatozoa themselves will not have to pass through the pumps to not harm the sperms by moving parts of the pump.

In a preferred embodiment, a spermatozoon containing fluid is caused to flow across the field of view while the sequence of images of a spermatozoon is acquired, and/or the field of view pictured in the images is illuminated bright enough to allow exposure times short compared to the time a spermatozoon substantially crosses the field of view pictured, and/or wherein the time span between two frames in a sequence of images is a fraction of the time a spermatozoon needs to cross the field of view pictured in the images.

The above suggested features of a preferred embodiment alone or in combination help to acquire non-blurred images. Typically, the spermatozoon containing fluid will flow through a microfluidic device from a reservoir, passing through or under an observation zone to an active sorting zone where a steering means directs a bolus of fluid containing one spermatozoon (or, in less preferred cases, several spermatozoa) to a reservoir selected according to an assessment. The observation zone in the present invention will be arranged in a manner allowing acquisition of images for the sequence; for example, the observation zone may have a transparent window that can be placed under the front lens of a microscope objective; the observation zone will typically be illuminated; where the observation zone has a transparent bottom, illumination can be effected by bright field-illumination; otherwise, other known in preferred illumination methods can be applied.

It will be understood that it would even be possible to arrange illumination devices such as LEDs, for example white light LEDs, alongside the observation zone. It will be understood by a person skilled in the art that it would be possible per se to integrate into the flow path of the microfluidic device passive sorting arrangements such as discussed above with respect to the prior art so as to obtain a preselection; it would also be understood that typically, pumps will be used to effect the flow of the spermatozoon containing fluid through the microfluidic device.

In certain instances, it will be preferred to dilute a sample of initially obtained ejaculate (or a preparation derived therefrom) so as to (further) reduce the number of spermatozoa simultaneously passing the observation zone. Such dilution can be effected inter alia when pumping the spermatozoon fluid through the microfluidic device, for example by pumping diluting fluid into a (pre-diluted or non-diluted) spermatozoa containing fluid. It will be understood that a sperm-containing sample might not only be obtained by ejaculation, but that it may also be obtained by biopsy; unless explicitly stated, although this will not make any difference in the context of the present invention, reference is usually had to an ejaculate as this is the more frequent way of obtaining the sperm-containing liquid. However, methods applied to obtain sperm may include inter alia penile vibratory stimulation, electrical ejaculation, sperm aspiration (TESA), percutaneous sperm aspiration (PESA), testicular sperm extraction (TESE), Microdissection TESE, and Microepididymal Sperm Aspiration (MESA). Also, reference will typically be made to the sample being an ejaculate rather than a preparation derived therefrom, although steps preparing the actual ejaculate for use will typically be carried out. In particular, sperm may be used that has been cryogenically stored and thawed for use.

Accordingly, in a preferred embodiment, it is also suggested that the fluid containing the spermatozoa to be assessed is pumped by a pump means through a microfluidic device for assessment, in particular across a field of view of an image sequence acquisition device. If this is done, it also is possible to operate pump means intermittently in a manner allowing for image acquisition during those intervals while no fluid is pumped across the field of view. This in turn will reduce the movement of a spermatozoon to be observed in the observation zone and will thus reduce blur in the images acquired, improving the assessment. It should be noted that microfluidic pumps can be operated at sufficiently high frequency and that in view of the flow geometry typical for microfluidic devices, the flow through the microfluidic device will remain laminar even where the flow changes it speed at a high frequency. Accordingly, there is no danger of damaging spermatozoa by shear forces; note that the channels provided in the microfluidic device for fluid flow will typically and preferably be significantly larger than the characteristic sizes of spermatozoa, for example having a cross section at least 3 times larger, preferably 5 times larger, in particular at least 10 times larger than the diameter of the sperm head. In a preferred embodiment, the fluid channel may be 5 times larger than the characteristic sizes of the sperm and will have a width of at least 15µm. Note that it usually is preferred to have a channel of rectangular or almost rectangular cross section so as to reduce optical distortions during imaging that would arise if the surfaces through which observation and/or illumination take place and hence in particular the upper and lower channel boundary walls are curved. Note that the surfaces may be slightly oblique to the optical axis to reduce reflections, but other than that should be perpendicular to the optical axis.

A person skilled in the art will understand that it might be preferable to adapt the microfluidic device used for sperm sorting to sperm of specific species, taking into account for example that the size of a human spermatozoon might differ from a bovine spermatozoon, a canine spermatozoon and so forth. In the preferred case of human spermatozoa selection, in particular human spermatozoa selection for ICSI methods, the microfluidic device will have an observation zone of more than 100 micrometres length, preferably 250 micrometres length or more, with a preferred channel length in the observation zone of 250 µm; in a practical example, a length of an observation zone of 250 micrometres has proved to be sufficient for observation. This must be compared to typical dimensions of a human sperm cell consisting of a flat, disc shaped head of 5.1 µm by 3.1 µm and a tail of about 50 µm length. It will be understood that the channel width or cross-section in the observation zone will usually be the same as in the fluid channel leading towards the observation zone.

Also, in a preferred embodiment, image acquisition is effected using a bright illumination of the field of view that can in particular be obtained by using a flash or strobe so that the spermatozoa are not subjected to a particularly strong illumination for a prolonged period; this is advantageous because it reduces the risk of damaging spermatozoa by illumination and the transfer of energy associated therewith. It will be understood that the time of bright illumination can be as short as 1/100 milliseconds, 1/200 milliseconds or even shorter such as 1/500ms or 1/1000ms. Note that the operation of LEDs is typically restricted by the power that can be dissipated; accordingly, short pulses that do not give rise to a particular severe heating are well acceptable for such illumination devices and hence, very short illumination times can be selected. As illumination pulses can be electronically generated in a very precise manner, the temporal spacing of frames can be highly controlled as the acquisition of images using a particularly bright illumination will not depend on the movement of a mechanic shutter or on the behavior of an electronic shutter of a CCD or CMOS sensor, nor will the time a final image actually was received in a storage be of particular importance; rather, the timing will only depend on the time of illumination of the observed object, that is the spermatozoa, and hence will only depend on the exact timing of the light pulses. Accordingly, it is possible to not only reduce blur but also reduce the uncertainties of acquisition time using a strobe or flashlight illumination.

In this context, it will also be understood that the time span between two frames -and thus the overall length of the sequences - will depend on the specific parameters used in a given arrangement such as the field of view, magnification, whether human or other sperm is assessed and so forth. Increasing the pump speed without changing the parameters will shorten the length of time available for image acquisition.

Enlarging the magnification will typically reduce the diameter of the field (or, where a microfluidic channel is observed, the length of the observation zone), so that the spermatozoon will pass through the observation zone in a shorter time, potentially reducing the number of images that can be acquired for a sequence; where this becomes critical because too few images for a sequence can be acquired, the flow rate would have to be reduced, thus potentially increasing the time needed for an assessment of a sample having a fixed volume such as 2 mL.

On the other hand, using a higher magnification might help in determining certain characteristics, in particular parameters describing the sperm head morphology using a higher magnification; accordingly, the number of frames needed for assessment could be reduced. From this, the average skilled person will understand that depending on the specific parameters examined, the species the spermatozoa stems from, the purpose of the assessment and so forth, the magnification optimizing throughput and or quality of assessment might vary. In a practical embodiment, magnifications between 100x and 400x have been preferred, with length of observation zones between 200 micrometres and 600 micrometres.

It should be noted that intermittently operating a fluid pump for pumping fluid across the field of view is possible with a high frequency, for example several kilohertz or several 10 kHz of switching between operation and non-operation. Also, instead of exciting a fluid pump for pumping fluid through a microfluidic device, valves could be used and intermittently opened/closed, the valves being arranged between a pressure reservoir and the field of view zone, for example between a reservoir of diluting solution pumped into the fluid reservoir from which the spermatozoa containing fluid is passed into the microfluidic device. Accordingly, the spermatozoa would not have to pass through the valves themselves.

In a preferred embodiment, the fluid containing the spermatozoa to be assessed is pumped through a microfluidic device for assessment by a pump means which can be operated in a first manner to provide a stream of fluid towards a first reservoir and which can alternatively be operated in a second manner to provide a stream of fluid towards a second reservoir, the first manner and the second manner being selected in response to a spermatozoon assessment signal and/or a spermatozoon selection signal.

In a preferred embodiment, the optical arrangement for observing the spermatozoa in the observation zone will allow for an automated focusing on a spermatozoon within the observation zone. One possibility to autofocus on a spermatozoon within the observation zone is to first identify the area or areas where a spermatozoon could be present; note that this might require to identify a spermatozoon even prior to having obtained best focus. However, identification of objects and images for example via artificial intelligence techniques frequently relies on significantly compressed data and is highly successful with such compressed data. This means that the highest spatial frequency usually only obtained in suitably focused images will not be necessary and that one or more images having but a resolution of for example 240 X 320 pixels instead of the megapixel resolution used for the actual assessment will be perfectly suitable for detecting areas where a spermatozoon is expected to be present. Based on such estimated areas, areas can then be determined which should be in focus. In a case where the subject such as a spermatozoon would not be moving, these areas would obviously be those where the object searched for was at the time of acquisition of the image later on e.g. compressed and analyzed by artificial intelligence techniques to determine the presence of the object. In a case where the object is moving, as should be the case with a suitable spermatozoon, the approximate speed of movement can be estimated from an initial sequence of a couple of images such as 2 to 4 very coarse images; note that the determination of focusing areas in a resolution-compressed coarse image will result in a plurality of uncompressed image pixels for which focus should be obtained; thus, the initial estimation of an approximate speed of movement for determination of a focusing area need not be particularly precise.

Once an area where focus is to be obtained has been determined, a plurality of possibilities to actually attain focus exists; if the microscope has a suitable photographic sensor with a sufficiently large number of phase detectors as known from conventional photography, those phase detectors that are within the selected areas can be identified and used for focusing, that is used e.g. to excite an autofocus drive physically changing the distance between a microscope objective and the observation channel. Also, focusing could alternatively and/or additionally be done inter alia by moving the entire microfluidic device towards the microscope objective or away from it; given that only fractions of a millimeter are needed for focusing adjustment, this can even be done at a surprisingly high speed. Furthermore, it should be noted that techniques other than phase detectors could be used as well. For example, exact focusing will result in contrast edges being sharper which is detectable by image analysis of images acquired. Accordingly, a plurality of possibilities exists to implement autofocus provided that the area where a focus should be obtained is correctly determined. Note that where a spermatozoon can be expected to not move significantly up or down within the channel, it is easier to maintain focus. Thus, in order to improve focusing, the fluid channel depth is preferably restricted very much in the same way as is fluid channel width.

For the sake of completeness of the disclosure, it is noted that techniques other than image analysis to determine areas in which focus should be obtained could be used; in this respect it is noted that a plurality of autofocusing techniques for microscopes are known and are appliable within the scope of the present invention. It is also noted that while autofocusing has become common in conventional photography, and while it has also been suggested to identify specific parts in an image such as the eyes of person to be portrayed, the applicant is not aware that autofocusing on spermatozoa as part of a sperm selection has been suggested before, leave alone by autofocusing on objects considered to be spermatozoa in view of image analysis techniques. This is considered inventive per se.

In a preferred embodiment, it is also suggested that the pump means comprises at least two separate pumps and the first manner of operating the pump means comprises operating (only) a first of the at least two separate pumps, whereas the second manner of operating the pump means comprises (only) operating a second pump of the at least two separate pumps and/or wherein the pump means comprises at least two valves, with a first valve of the at least two valves being opened for operating the pump means in the first manner whereas for operating the pump means in the second manner, a second valve of the at least two valves is opened. Using two different pumps for steering the fluid to a selected one of two reservoirs is particularly simple with respect to both the electronics to be used and the design of the microfluidic device. Note however that other steering means could be implemented, more than two different destination reservoirs could be provided and so forth.

In a preferred embodiment, it is also suggested that a fluid containing the spermatozoa to be assessed is caused to flow thru a microfluidic device in a manner such that during at least one of image acquisition and sorting, a laminar flow environment is provided for the spermatozoa and/or such that the spermatozoa are maintained free of external fluid forces during at least one of image acquisition and sorting. As indicated above this is advantageous because it reduces shear stresses on the spermatozoa; the invention allows to not only observe the spermatozoa optically without inducing damaging shear stress, but to also effect sorting in a shear stress unaffected manner.

Accordingly, a spermatozoon sorting method is suggested based on the methods disclosed above, comprising the generation of a spermatozoon assessment or selection signals for a plurality of spermatozoa and further comprising sorting spermatozoa into one of at least two different sorting reservoirs in response to the assessment signal or selection in accordance with one of the methods disclosed above. It will be understood from the above that it is preferred to surround the spermatozoa in a bolus of fluid sufficiently large so as to reduce or completely avoid any damage due to contact with surfaces, shear stress and so forth. Surrounding the spermatozoa in a sufficiently large bolus of fluid is possible by properly designing the size of microfluidic channels.

Given that the spermatozoa will not be affected by shear stress in a microfluidic device of the invention, it also is suggested that fluid containing the spermatozoa is passed through a microfluidic device for image acquisition and/or sorting and that the spermatozoa in the fluid are diluted and/or singled out prior to image acquisition and/or sorting.

From the above, it will be understood that one aspect of the disclosure considered novel and inventive relates to a microfluidic spermatozoa sorting device comprising at least three fluid reservoirs for spermatozoa containing fluid, a microfluidic flow arrangement between a first of the three fluid reservoirs and the at least other two fluid reservoirs comprising a fluid steering means for steering in a steering zone the spermatozoon containing fluid from the first reservoir to one of the at least two other fluid reservoirs, the microfluidic flow arrangement comprising a transparent section allowing acquisition of the sequence of images of spermatozoa, the transparent section being arranged at a distance so far away from the steering zone that that the sequence of images can be analyzed by a method as described above prior to the spermatozoon reaching the steering zone, the sorting device being adapted to apply a signal generated according to a method of one of the preceding claims to the fluid steering means for controlling the reservoir into which a spermatozoon is steered. It will be understood that other aspects of the disclosure might relate to a microfluidic spermatozoa sorting device having in particular no reservoir for selected sperm; for example, a laboratory apparatus according to the invention for use in an ICSI procedure might comprise a microfluidic spermatozoa sorting device with a microfluidic flow arrangement for causing a microfluidic flow of spermatozoa from a reservoir through an observation zone and into a steering zone, the laboratory apparatus having image acquisition means for acquiring sequences of images of spermatozoa while the spermatozoa are in the observation zone, a computer assessment stage for assessing the sequences of images both with respect to motility and morphology of spermatozoa and for selecting spermatozoa in view of the assessment, the laboratory apparatus being further adapted to steer selected spermatozoa within the steering zone into a direction reserved for selected spermatozoa.

The invention will now be described by way of example only and with respect to the drawing which shows in:
- Figure 1:: a diagrammatic representation of an embodiment of the present invention;
- Figure 2a:: a perspective view of components of the sperm picking system of figure 1
- Figure 2b:: details from the embodiment of figure 2a
- Figure 3:: a schematic representation of a first sperm picking system;

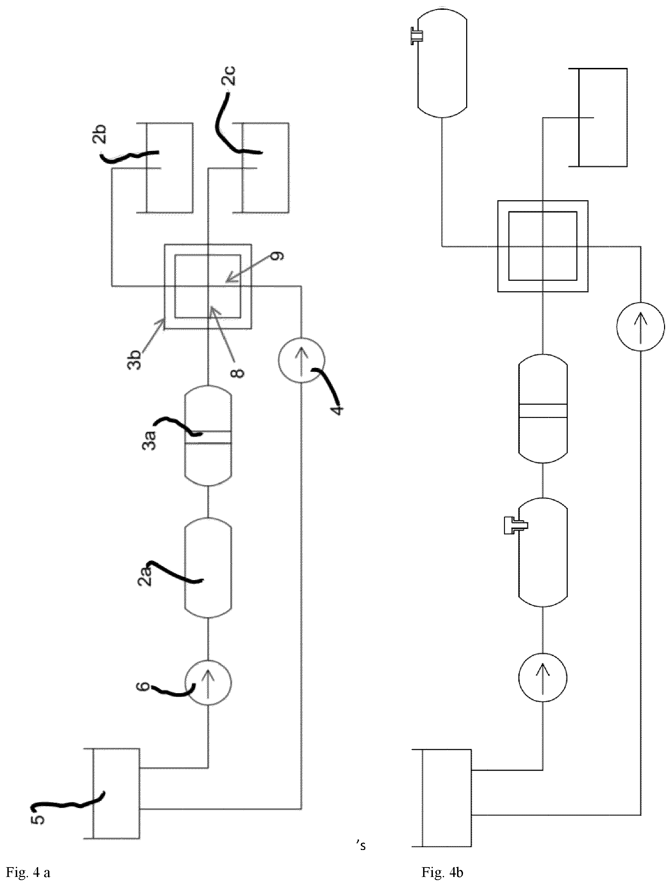

- Figure 4:: a schematic representation of another embodiment of a sperm picking system;
- Figure 5a:: a schematic drawing of an embodiment of a sperm picking system using cyclic sperm picking during a selection phase;
- Figure 5b:: a schematic drawing of the embodiment of figure 5a during a recycling phase
- Figure 6:: an illustration of sperm selection in the microfluidic chip.

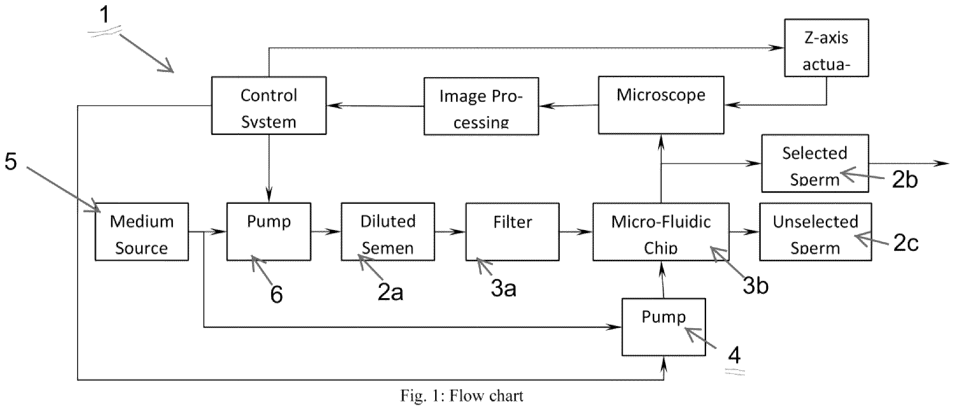

According to figure 1, a microfluidic spermatozoa sorting device generally designated by reference numeral 1 comprises at least three fluid reservoirs 2a, 2b, 2c for spermatozoa containing fluid, a microfluidic flow arrangement between a first 2a of the three fluid reservoirs and the at least other two fluid reservoirs 2b, 2c comprising a fluid steering means 4 for steering in a steering zone 10 the spermatozoon containing fluid from the first reservoir 2a to one of the at least two other fluid reservoirs 2b, 2c, the microfluidic flow arrangement comprising a transparent section allowing acquisition of a sequence of images of spermatozoa, the transparent section being arranged at a distance so far away from the steering zone that before the spermatozoon reaches the steering zone, the sequence of images can be analyzed by an automated computerized method that will be described hereinafter, the sorting device being adapted to apply a signal generated according to a method to the fluid steering means for controlling the reservoir into which a spermatozoon is steered.

The microfluidic spermatozoa sorting device of figure 1 typically serves to select spermatozoa for an in vitro-fertilization although other uses would be possible, for example to isolate for research purposes spermatozoa having a specific set of deficiencies; the transparent section together with suitable microfluidic channels forms part of a microfluidic chip 3b which in turn forms part of the microfluidic spermatozoa sorting device of figure 1. The microfluidic chip can be produced e.g. by rapid manufacturing or by etching. The microfluidic chip may be a reusable or, more often, a disposable item and may be specifically adapted for use with the present invention, for example with respect to the dimensions and arrangement of fluid channels, connections to valves and reservoirs and so forth. Applicant specifically reserves the right to claim and patent such disposable or re-usable item.

The transparent section is arranged such that it allows to observe spermatozoa in an observation zone using a suitable microscope; accordingly, the transparent section will have upper and lower boundary surfaces that are plane and are perpendicular to the optical axis of the microscope. Having plane boundaries minimizes optical distortions of the observation zone. Having planes perpendicular to the optical axis of the microscope will reduce spermatozoa movement in the direction of the optical axis, thus reducing the introduction of errors in the estimation of motility and also reducing the need for refocusing. It will be understood that the microfluidic chip can be disposable; the same obviously holds for any other parts of the microfluidic channels that comes into direct contact with spermatozoa containing fluid, thus significantly reducing the risk of contaminating samples. Downstream of the observation zone, an interconnection channel 8 leads to the steering zone.

The microscope will have a magnification sufficient for determining topological properties of spermatozoa in a manner sufficient for distinguishing spermatozoa that should not or cannot be used for fertilization from spermatozoa that are to be selected for fertilization. For human spermatozoa, a magnification between 200-fold and 400-fold has been found to be most preferable.

The microscope is adapted to acquire sequences of digital images of objects in the field of view, in the embodiment with a frame rate 24 frames per second. The microscope camera in the embodiment shown has a resolution of 1920X1080 pixels which has been found to be sufficient to determine a large number of parameters with a sufficient precision; however, it can be estimated that given general progress in imaging technologies, resolutions will increase in the future. In the embodiment shown, the observation zone is about 250 micrometres long; with an average speed of movement of healthy, usable spermatozoa across the field of view and typical pumping speeds that allow selection of a suitable number of sperms in an acceptable time a spermatozoon will typically be within the observation zone for approximately 3 to 10 seconds. This allows acquisition of typically more than 70 frames; however, the microchannel must be somewhat larger than the diameter of the sperm head, thus allowing the sperm to move either closer to the top surface of the microfluidic channel or closer to the bottom surface thereof. Accordingly, several of the 70 frames, in particular some of the initially acquired frames, will be used to initially adjust the focus in an automated manner.

Also, it may be necessary to select those frames of a sequence where the respective topological parameters of the spermatozoa considered in a selection pass can be determined best, for example because a given spermatozoa is correctly oriented in only some of the images and/or some of the images are sharper than others. The arrangement is provided with an image evaluation stage having sufficient processing power to process all usable images in a sequence and to calculate parameters improved with statistical methods such as averaging, deletion of outliers and so forth. Note that while good results could be obtained even with a lower frame rate, the frame rate indicated has the additional advantage that observing the video stream is less tiring.

The microscope will be provided with an autofocusing arrangement. In more detail, the microfluidic chip is placed on an actuated stage that can be moved in the "z" direction, that is the direction of the optical axis of the microscope; the actuation allows for a minimum travel of 50 micrometres in the embodiment shown within 1/25-th of a second. Note that an additional coarse adjustment is also provided so that the minimum travel allows to focus on any object within the observation zone of the microfluidic chip once coarse adjustment is achieved. In the embodiment shown it is possible to allow for an automated focusing on a spermatozoon within the observation zone. To this end, first, an image is compressed to the size of 240 X 320 pixels instead of the megapixel resolution. In the compressed image, areas are detected using artificial intelligence techniques where a spermatozoon is likely to be present. Note that rather than using artificial intelligence techniques, a motion history image could be established for a couple of compressed images and the areas could be identified where motion is present according to the motion history image. From these areas, sensor areas are preselected where an image is to be sharp; then, a series of images is acquired while adjusting focus by micro-translations of the z-stage until the image in the areas preselected is sharp.

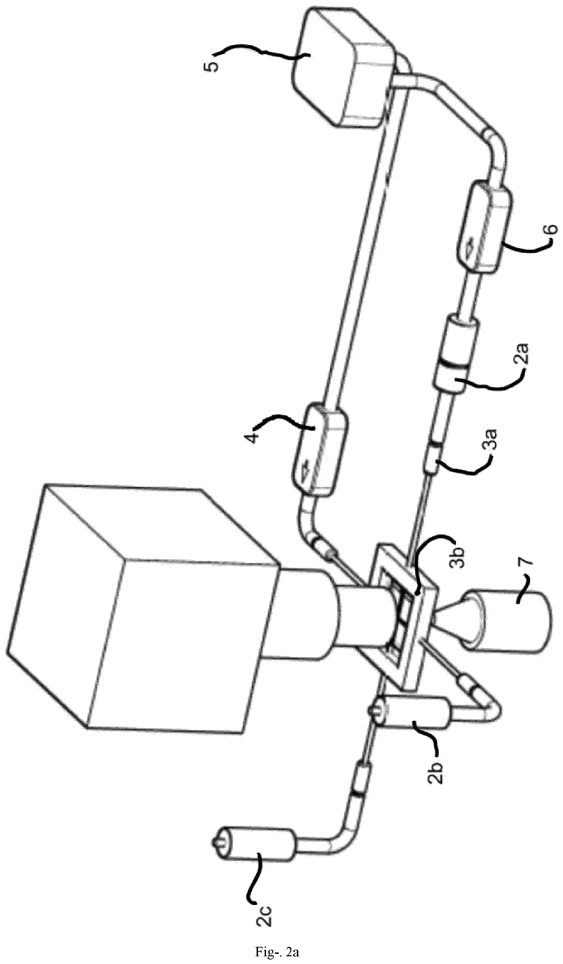

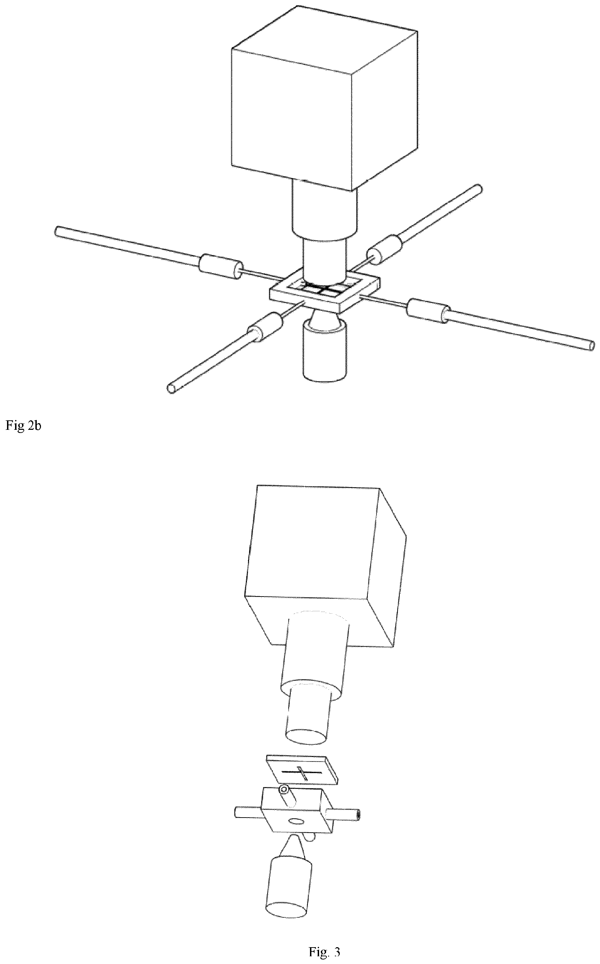

In the embodiment shown, the observation zone is illuminated from below using a conventional thru-light (or bright-field) condenser, as can be seen in particular in figure 2 where the condenser 7 is shown below the microfluidic chip 3b. The condenser can be any conventional illumination device, although in a preferred embodiment, light pulses are emitted that are short enough to prevent blur from movement in the images; a pulse duration of 1/500sec has been found to be short enough and can be well synchronized to the exposure of frames.

The reservoir 2a in figure 1 will contain a preparation or sample of semen obtained by biopsy or as an ejaculate. The semen in reservoir 2a is usually pre-filtered and pre-diluted to a sperm density in the fluid that allows to observe most spermatzoa in the observation zone as single spermatozoa. Fluids that can be used for dilution are well known in the art and need not be described here: one commercially available fluid is a multipurpose handling medium complete with Gentamicin for handling sperm, oocytes, and embryos in a stable environment and available from Irvine scientific. It might be preferred if the dilution of the initially obtained sample is such that occasionally, a spermatozoon in the observation zone is observed while one or a few other spermatozoa are present there as well. In this manner, a dilution too high can be avoided. It will be understood that a very high dilution requires a longer time of sorting, thus reducing throughput, while a very low dilution will result in a plurality of spermatozoa being simultaneously present in the observation zone, which makes selection of spermatozoa difficult and thus has adverse effects on the results obtainable. Also, it will be understood that a pre-filtering or other preconditioning of the spermatozoa containing fluid can be effected. Furthermore, it will be understood that the spermatozoa may come from a sample that has been stored at low temperatures for a prolonged time before use, for example in liquid nitrogen. In such a case, the fluid will have been returned to a suitable temperature before assessment.

The reservoir 2a has an outlet for spermatozoa containing fluid and an inlet for pressurizing, the reservoir 2a being constructed such that the application of pressure results in spermatozoa-containing fluid flowing through the outlet.

The inlet to reservoir 2a is connected to the outlet side of a pump 6 which at its pump inlet side draws liquid medium from a medium reservoir (or medium source) 5. The pump 6 can be controlled by a control system in a manner such that flow can be switched on or stopped at a high frequency, in the present embodiment 15 kHz. It will be understood that in view of the preferred high frequencies of switching flow on and off, the presence of a compressible medium in reservoir 2a is to be avoided; accordingly, in the preferred embodiment, no ambient air or other gas will be present in the reservoir 2a.

The medium in the medium source 5 can be the same as mentioned above used for diluting a semen sample and known in the art.

The pump 6 is adjustable such that flow of fluid through the microfluidic device results in an average speed of usable spermatozoa that allows to identify and select within an acceptable time a sufficient number of sperm even in samples containing a very low percentage of spermatozoa usable for ICSI procedures. For example, where a sample with 400,000 sperms per milliliter contains about 0,1% usable sperms (compared to 4% in a sample from a normal healthy male) and about 100 spermatozoa shall be selected, it is necessary to inspect a total number of spermatozoa of approximately 100,000. This corresponds to an overall length of spermatozoa to be inspected of about 100 mm and if this length is to be analyzed within one hour, the velocity would only need to be between 25 and 30 µm per second. Note that the volume that needs to be pumped in to the example will correspond to 0,0025 mL of undiluted sperm sample or, after a 1: 10 dilution, to a volume of 0,025 milliliters. This throughput can easily be achieved with conventional microfluidic pumps. Also, it will be understood that even for conventional frame rates such as 24 frames/second, at the velocity indicated, the time a spermatozoon needs to pass the observation zone of about 250 micrometres width will be sufficient to acquire more than hundred frames which is more than enough for initial focusing and subsequent analysis both of motility and morphology. As this number of frames is significantly higher than necessary, it is possible to increase the pumping speed, for example to 80 µm/s.

The outlet of reservoir 2a is connected to the input side of a fluid filter 3a which in turn is connected at its outlet side to a first input 3b1 into microfluidic chip 3b, in particular the sample input opening of the channel which leads to the transparent section. The fluid filter 3a has a pore size adapted to prevent particles significantly larger than a single sperm or aggregates of too many spermatozoa from reaching and subsequently clogging the microfluidic chip 3b.

The microfluidic channels in the microfluidic chip 3b leading to the transparent section in the embodiment shown in figure 1 have a cross-section of 50 micrometres width and will also have a depth of 50 micrometres.

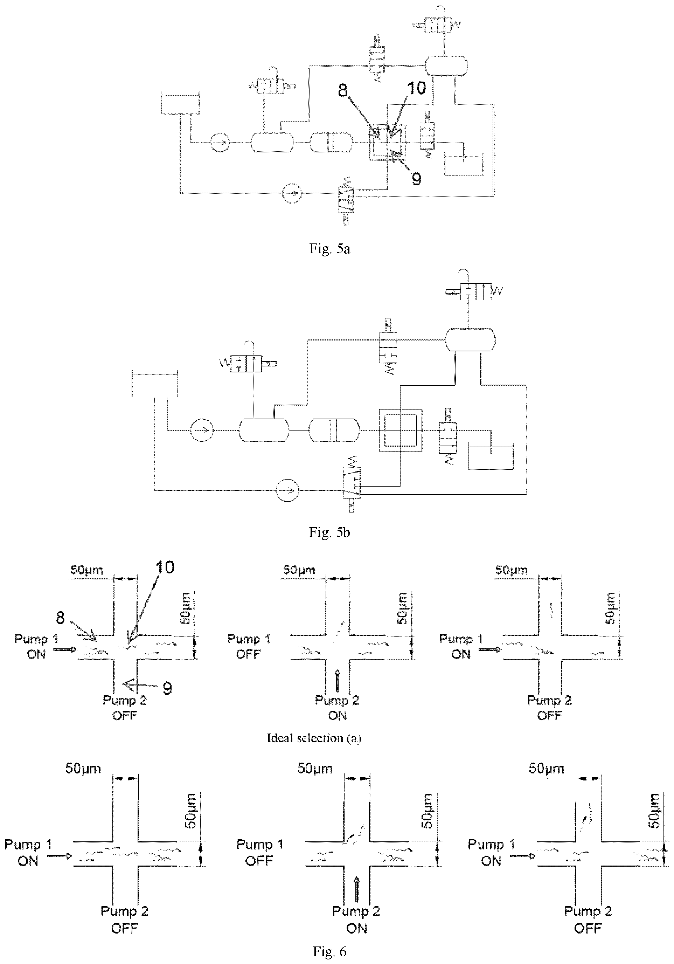

Microfluidic chip 3b has a further input 3b2 connected to the output side of a steering pump 4 which pumps medium from medium source 4 into a second channel 9 of the microfluidic chip 3b. The second channel 9 leads to the steering zone in a direction perpendicular to the direction of interconnection channel 8 as can be seen in particular in figure 6. As can be seen in figure 6, opposite the side where the interconnection channel to the observation zone enters the steering zone, a first fluid outlet into a channel allowing fluid transfer into the second reservoir is provided. Also, opposite the side where the second channel 9 enters the steering zone, a second fluid outlet into a channel allowing fluid transfer into the third reservoir is provided. It will be understood that it is not necessary that the entire path and the second and third reservoirs constitute part of the microfluidic chip; rather, it is possible that a tubing is provided including for example flexible Teflon tubes leading to the second and third reservoir respectively.

The steering zone 10 is arranged such that if the steering pump 4 is switched on, steering medium pumped into the steering zone will entrain any spermatozoa in the direction of the flow of the steering medium. (It will be understood that the steering pump 4 draws fluid from the medium source 5, so that referring to the fluid that the steering pump 4 is pumps into the steering zone to be "steering fluid" is merely for better understanding.)

Steering pump 4 can be switched on and off at a high frequency by a control arrangement; in the embodiment shown, switching frequencies of up to 15 kHz are possible. The control arrangement for steering pump 4 is adapted to also control pump 6, in particular in a manner such that pump 6 will have to be switched off during the phases where switching on of steering pump 4 is allowed. In this manner, spermatozoa that have reached the steering zone through interconnection channel 8 will either continue along a more or less straight line in case pump 4 remains switched off, so that they will reach the outlet from the steering zone leading into reservoir 2c; alternatively, if pump 4 is switched on, the spermatozoa will be entrained with the fluid entering the steering zone from a direction orthogonal to the axis of the interconnection channel and will flow through the second fluid outlet let into the channel leading to the third reservoir 2b.

The control is adapted to operate the steering pump 4 for an interval that is sufficient to pump a volume of steering fluid into the steering zone larger than the steering zone volume; accordingly, a bolus of fluid surrounding a single spermatozoon (or, depending on the dilution of the sperm sample, a small number of spermatozoa) will be pushed into the channel allowing fluid transfer into the third reservoir. It will be understood that the distance a bolus is pushed into the channel will be such that within the time expected to pass until the next bolus is pushed into the same direction, even a normal, highly motile sperm will not be able to return to the steering zone. Under typical conditions in the embodiment shown, this will be the case if the bolus is pushed 120 micrometres into the respective output channel. It should be understood that although this length is not considered to be restrictive, lengths of 100 µm have proven to prevent most spermatozoa from drifting or moving back while a length of 200 µm or more does not result in better protection against return from protection from moving back. (Note that the possibility exists in a preferred embodiment, not shown, to direct medium free of spermatozoa into the fluid channel so as to separate one bolus from the next.)

It will be understood from figure 1 that preferably, spermatozoa selected are steered into the second reservoir 2b by switching on pump 4, while spermatozoa not selected are allowed to propagate into the channel leading to the third reservoir 2c.

Accordingly, by selecting the operation state of the steering pump means is, the direction into which a spermatozoon in the steering zone will be moved can be selected.

It will be understood that in the embodiment shown, the steering zone is different from the observation zone, but that it would be possible to combine the observation zone and the steering zone.

The control arrangement to control pump 6 and steering pump 4 is receiving a signal from an image analyzing stage adapted to analyze the images of a spermatozoon in the observation zone.

In the image analyzing stage, image analysis is effected by determining several parameters characterizing the sperm in response to one or more of the images acquired.

Reference is made to the following documents indicating the importance of several such parameters: "World Health Organization reference values for human semen characteristics " by Cooper TG, Noonan E, von Eckardstein S, Auger J, Baker HW, Behre HM, Haugen TB, Kruger T, Wang C, Mbizvo MT et al. in Hum. Reprod. Update 2010;16:231-245; "World Health Organization Laboratory Manual for the Examination and Processing of Human Semen", WHO Geneva: World Health Organization, 2010; United States patent application US 15/420,445 "Interferometric system and method for use with biological cells and organisms including sperm" by inventors Shaked NT, Girshovitz P, Barnea I, Balberg M, Mirsky S, Eravuchira PJ, inventors; TECHNOLOGY INNOVATION MOMENTUM FUND (ISRAEL) LP, assignee. Reference should also be had to the "Atlas of human sperm morphology evaluation", by T F Kruger_ Daniel R Franken, -Taylor & Francis (2004), in particular to pages 52 - 73 therein.

Regarding relevant parameters that can be assessed in an image analysis, the following is noted.

A head area can be determined based on the number of pixels the head fills out in an image; it will be understood that this number will depend on the magnification and that accordingly, it is preferable to use a constant magnification in the determination of this and other parameters.

A total volume of the cell head can also be determined; while acquiring the sequence of images, the sperm is likely to rotate and thus is likely to be observed from different directions so that from the head areas observed for different orientations, a volume can be estimated.

A width of the head area and a length of the head area can be determined; note that either the maximum/minimum length can be determined in a sequence of images and/or an average maximum/minimum value of the length and/or the width of the head area.

A volume of vacuoles within the cell head, and their volume relative to the head volume can be determined; note that the volume of vacuoles can basically be determined in the same manner as the total volume of the cell head.

A parameter can be determined indicating the total number of pixels around the boundary of each region in the image.

The 2D shape of the sperm head can be analyzed, assessing for example a form factor such as (4^{∗}pi^{∗}Area excluding holes)/ (Perimeter^{∗}Perimeter); this parameter would equal 1 for a perfectly circular object; a roundness calculated e.g. as (4^{∗}Area including holes)/pi^{∗} (Maximum diameter^{∗}Maximum diameter); an aspect ratio such as e.g. the ratio (Major axis)/(Minor axis) or the ratio (width of head)/(length of head); an effective diameter calculated e.g. as (Area including holes/pi) ^{∗}2; a circular degree calculated e.g. as pi^{∗}(Major axis)/4^{∗}(Area excluding holes); a circularity ratio calculated as (4^{∗}pi^{∗}Area including holes)/ (Perimeter^{∗}Perimeter); a thin degree calculated e.g. as (Maximum diameter)/(Pattern width); a compact aspect ratio calculated e.g. as ((4/pi)^{∗}(Area including holes)))^{1/2}/(Major axis); an elongation calculated e.g. as: (Perimeter^{∗}Perimeter)/(Area including holes); a roughness calculated e.g. as (perimeter^{∗}perimeter)/(4^{∗}pi^{∗} (area excluding holes)); a degree of circularity calculated e.g. as: 2^{∗}(pi^{∗}(Area including hole))^{1/2}/Perimeter; a relative position of head span determined as a relative distance of the position along the length of the head at which the width of the head is maximum; a longitudinal asymmetry measure indicative of the longitudinal asymmetry of the head can be calculated by first dividing the head by a line starting from the point at which the midpiece connects to the head and leading to the farthest point from this midpiece point located on a best-fit ellipse fitted to the head. Using this line as a symmetry axis, the resulting halves of the head can then be overlaid by folding them along the axis of symmetry. Counting the number of nonoverlapping pixels and subsequently dividing this number by the total number of pixels in the head ("head area"), results in a longitudinal asymmetry measure.

Also, an anterior Area Distribution can be considered, this parameter comparing the head to a perfect ellipse possessing the same length and width as the head. The comparison is effected by subtracting the model ellipse from the head image and by then summing up the remaining number of pixels in the anterior half of the image; this number is then divided by the area of the model.

A head non-ellipsity can be determined by first dividing an image of the head into two parts along its length, using the point at which the width of the head is at a maximum for dividing the image; then, the anterior part is mirrored in order to produce an elliptical region; thereafter, an image of a best-fit ellipse is calculated and subtracted from the head image. The number of deviating pixels can again be summed; dividing this sum by the head area is used to normalize this parameter. Another parameter, the anterior distension ratio, can be calculated by counting only the number of "negative pixels", that is those pixels where the head is smaller than the model ellipse; again, this number can be normalized relative to the area. Also, a posterior distension ratio can be calculated using basically the same process described for the anterior distension ratio, however in this case, summing up the number of "positive" pixels and normalizing by head area; a net distension ratio can be calculated then as the difference between the anterior distension ratio) and the (posterior distension ratio.

An ellipse structural similarity index (SSIM) can be calculated using an elliptical mask having the same length and width as a reference image head.

Furthermore, the following parameters can also be obtained by image analysis of the head and other parts of a spermatozoa: Solidity as a measure for the proportion of pixels in the convex hull that are also in the object, i.e. Object-Area/ConvexHullArea. This parameter is equal to 1 for a solid object having no holes or a concave boundary and is smaller than one for an object having holes or possessing a convex/irregular boundary.

The "extent", a measure of the area of sperm image pixels relative to the area of a bounding box surrounding the sperm, can be calculated.

The "eccentricity" as a parameter relating to a model ellipse having the same second-moments as the region of the head image. The eccentricity is defined as the ratio of the distance between the foci of the ellipse and its major axis length. Note that the eccentricity of a perfect circle is 0, while the degenerate case of a line segment has an eccentricity value of 1.

The Euler number as a measure of holes in the sperm head can be calculated.

A MajorAxisLength and a MinorAxisLength can be determined as the length (in pixels) of the major or minor axis respectively of the ellipse that has the same normalized second central moments as the image region of the sperm head.

A compactness can be defined referring to the mean squared distance of the object's pixels from its centroid, divided by the area for normalization. A filled circle will have a compactness of 1, with irregular objects or objects with holes having a value greater than 1.

A MinFeretDiameter and a MaxFeretDiameter can be defined with a Feret diameter being the distance between two parallel lines tangent on either side of the sperm head, very similar to using a caliper on a real object. The distance between the 2 parallel tangent lines will depend on where the tangent lines are placed on the sperm head image; accordingly, the maximum and a minimum distance of these lines will be observed depending on the orientation of the lines; the minimum and maximum Feret diameters are then defined as the smallest and largest possible diameters, rotating the imaginary calipers along all possible angles.

Regarding the maximum distances of pixels of the sperm image to the closest pixel outside the sperm, a "MaximumRadius" can be defined as the maximum distance any pixel in the sperm image has to the closest pixel outside the sperm and a MedianRadius can be defined as the median of those distances.

An approximation of the sperm contour can be calculated based on a sequence of polynomials that are orthogonal on a unit disk; suitable polynomials are known as Zernike polynomials. The corresponding coefficients of the sequence can be used as parameters; where the polynomials are Zernike polynomials, the coefficients constitute the so-called Zernike shape features.

It is noted that it is possible to consider not only the shape of the spermatozoa imaged, but that in particular, also the intensity (or the gray value) of pixels in the images can be assessed, provided of course that illumination and exposure conditions are kept constant and/or that a normalization can be effected, for example using a background without objects. In more detail, a coefficient of variation of intensity within a ring can be calculated for rings placed for example around the centroid and weighted centroid of the sperm head or placed around other specific parts of the sperm; a granularity can be calculated and a TotalIntensity representing a sum of all pixel intensity values can be calculated. Mean and median intensity of pixel intensity values within the sperm head image can be calculated; standard deviation and median absolute deviation (MAD) of pixel intensity values can also be calculated.

Then, Minlntensity and MaxIntensity values of the pixels within the sperm image or sperm head image can be calculated, and percentile intensities, for example a LowerQuartileIntensity can be calculated or an UpperQuartileIntensity indicating the intensity value of the pixel for which 75% of the pixels in the object have lower values.

Also, several parameters relating to the so-called texture of a sperm could be determined, using for example Haralick texture features. As is known per se in the art, Haralick texture features are derived from the co-occurrence matrix, which contains information about combinations of intensities in neighboring pixels. In order to determine this co-occurrence matrix, the image is quantized into eight intensity levels. Accordingly, there exists 8x8 possible combinations of intensities of 2 neighboring pixels. The 8x8 co-occurrence matrix used for the determination of the Haralick features can thus be formed by counting how many pixel/neighbor-pixel pairs belong to each of the 8x8 intensity combinations. Based on this 8x8 co-occurrence matrix, a plurality of parameters can then be derived as is known per se in the art.

In more detail, an angularSecondMoment can be calculated as a measure of image homogeneity such that a value of 1 is obtained for a uniform image, while a higher value of this feature indicates that the intensity varies less in an image. Contrast can be calculated as a measure of local variation in an image such that a value of 0 is obtained for a uniform measure, while a high contrast value indicates a high degree of local variation. A correlation can be calculated as a measure of linear dependency of intensity values in an image in a manner such that for an image having large areas of similar intensities, the correlation is much higher than for an image with noisier, uncorrelated intensities. In particular, the correlation can be calculated such that it has a value of 1 or -1 for a perfectly positively or negatively correlated image. A variance as a measure of the variation of image intensity values can be calculated in a manner such that for an image with uniform intensity, the texture variance would be zero. An InverseDifferenceMoment can be calculated as a representation of image contrast such that it has a low value for inhomo-geneous images and a relatively higher value for homogeneous images. A SumAverage can be calculated such that it is the average of the normalized grayscale image in the spatial domain and a SumVariance can be calculated such that it represents the variance of the normalized grayscale image in the spatial domain. Furthermore, a SumEntropy can be calculated such that it represents a measure of randomness within an image and an entropy can be calculated as an indication of the complexity within an image, in particular such that a complex image produces a high entropy value. Furthermore, a DifferenceVariance can be calculated representing the image variation in a normalized co-occurance matrix. Then, a DifferenceEntropy can be calculated as another indication of the amount of randomness in an image, and several info Measure of correlation can be calculated.

Where a phase contrast microscope is used, several optical path differences can be assessed; for example a mean optical path difference (OPD) of each head region can determined; in addition, it is also possible to determine other parameters as suggested by WHO for assessing sperm morphology, for example a midpiece width; a midpiece length; a tail length; a tail form; a head form and the presence or absence of cytoplasmic droplets.

A maximum value of normalized 2-D cross-correlation can be calculated using a predetermined number of images from of heads of cells possessing good head shapes e.g. as determined by a trained clinician or referring to a database established by storing images of spermatozoa that have been successfully used in fertilization; from such images, an average optical path difference (OPD) image for good sperm heads can be determined and used as a template. The maximum value of normalized 2-D cross-correlation between the tem-plate and head image is then calculated.

Regarding this image analysis, it will be understood that many of these parameters, analysis is fast and does not require a high processing power. Also, it will be understood that most frequently, only a selection of the above mentioned parameters will be calculated to assess whether any given spermatozoa should be selected or discarded. For the embodiment shown, only some parameters are used in the first pass, namely the size of the head, ovality, size of the acronym, the angle of the mid piece and tail; while this list is non-comprehensive, good results have been achieved based on this small set of parameters. In a second step, it is possible to check for vacuoles within the head of the sperm.

Then, it is well possible that certain parameters also depend on the specific characteristics of the image acquisition.

It will also be understood that the values of parameters spermatozoa must have to be selected might vary over time, depending on the progress of scientific knowledge. Also, these parameters will vary depending on the devices used for acquiring the images and may also depend for example on the settings of image processing parameters. For example, the Haralick texture features assessing the distribution of intensity levels might depend on the linearity of a contrast curve and hence be machine-dependent. Furthermore, the possibility exists that certain spermatozoa should be selected or deselected in view of a combination of several parameters rather than in view of single parameters. Accordingly, no specific boundaries the sperm should have to be selected are indicated for the respective parameters.

However, the present invention suggests to not only determine parameters in a single image acquired of a spermatozoon. Also, the present invention does not restrict itself to only calculate improved parameters by evaluating a plurality of single images of one and the same spermatozoon, for example by calculating the spermatozoon head area for each image in the sequence and plan averaging over the head areas, although this obviously can be done and is preferable. Rather, the present invention suggests to evaluate from the sequence of images not only morphologic parameters, but also assess motility of each single spermatozoon.

In more detail, several parameters relating to the motility can be assessed. First, an assessment can be made on the overall movement of a spermatozoon; obviously, as sperm -containing liquid is pumped through the microfluidic device by pump 6, some movement will occur even for non-viable spermatozoa. Superimposed on this "background movement" will be a movement caused by the spermatozoon itself, in particular resulting from the movement of the sperm tail. The movement of the sperm tail of healthy spermatozoa will correspond to specific patterns that can be identified in the sequence of images, using for example motion history image (MHI) techniques. Also, motion history images could be evaluated using artificial intelligence techniques. It is possible to identify and assess the changing shape of the tail, to assess the resulting (rotational) movement of the head and so forth. Accordingly, assessing motility is not restricted to merely determining an overall movement.

Again, similar to the parameters describing morphology/topology of the sperm and in particular of the sperm head, one or a plurality of parameters relating to motility can be used to determine whether or not the movements observed are indicative of a viable spermatozoon that should be selected for fertilization.

Then, a decision on whether or not a given spermatozoon should be selected can be made based on both motility and morphology. In a most simple case, spermatozoa could be deselected if either parameter indicates that minor problems might exist. Where an extremely low number of viable spermatozoa are found in a sample, it might however be necessary to select spermatozoa as long as at least an overall evaluation taking into account both motility and morphology still is acceptable. From this, it can be seen that depending on the overall quality of the sample, the thresholds of the sperm parameters might have to be adjusted. It will be understood that the thresholds could be dynamically adjusted, in particular lowered if the assessment of more and more spermatozoa of a sample does not yield a useful selection rate otherwise.

Even though a proper selection can highly increase the success rate of fertilization, it is reasonable to admit that success in an ICSI fertilization at least currently is not obtained with every single egg, despite very careful spermatozoa selection. This however allows to set up a database wherein image sequences of spermatozoa selected for ICSI fertilization and information relating to the success of the respective fertilization are combined.

Once such a database has become comprehensive enough, that is once a sufficient number of image sequences and information relating to success or failure of using the respective spermatozoa for fertilization has been acquired, it will be possible to use such information for improving the selection. It should be understood that even though certain parameters have been described above as being useful in spermatozoa selection, it will not be absolutely vital to determine the exact parameters once a sufficiently large database has been acquired. Rather, artificial intelligence techniques can be used; here, the image sequences in the database together with the information relating to the success or failure of a given spermatozoon can be used for training of an artificial intelligence model. Accordingly, it is expected that once a sufficiently large database has been established, sequences of images of each spermatozoon will be assessed using an artificial intelligence model trained in view of sequences of images of spermatozoa known to fail or succeed in fertilization. Once this is done, it will not be necessary to determine single parameters any longer. Note that artificial intelligence methods are well suited for image analysis. Sequences of images could be analyzed using the same methods by combining of frames of the sequence into one combined image and/or by first determining additional information such as a motion history image or a plurality of images descriptive of moving objects within a sequence; reference is specifically made to techniques developed for the determination of movement in video sequences.

It will be understood that analysis of the image sequences may be effected either onsite of the microfluidic device, for example in a laboratory where the microfluidic device, pumps, microscope and so forth are placed or close by to the such a laboratory, for example to reduce noise in the laboratory due to ventilators in computers running and so forth.

However, it is also feasible to transfer sequences of images of a given spermatozoon via e.g. a local area network or via a UMTS or G4, G5 and so forth wireless connection to a server and to effect the analysis there. Then, the result of the analysis, that is a decision whether or not to select a spermatozoon observed in the observation zone and depicted in the sequence of images transferred to the server, can be transmitted to the laboratory. Where a sufficiently broadband connection is provided, the time necessary for initially transmitting the sequence of images to the server, for processing the sequence of images on the server and for transmitting the decision whether or not to select the spermatozoa are observed is small compared to the time a spermatozoon needs to pass through an interconnection channel of acceptable length. Accordingly, it is possible to execute the computerized steps of sperm selection for a real-time decision on a remote server. Nonetheless, where autofocusing of the microscope relies on an evaluation of the first images acquired of each spermatozoon, it may be preferred to have at least the assessment for autofocusing on site. Note that where autofocusing can be effected without analyzing images, for example using "conventional" microscopic autofocusing techniques known in the art, not even such local processing will be necessary. Also, it can be understood that even where a local assessment of image sequences is effected, a central database including image sequences of selected spermatozoa together with information with respect to the success of fertilization using the selected spermatozoa can be established by receiving the respective information from a plurality of laboratories. This is particularly useful in cases where identical or at least similar set-ups are used.

The device of the present invention can be used in manner implementing a computer-assisted spermatozoon assessment method comprising feeding spermatozoon related digital data into a computerized spermatozoon assessment stage and automatically generating by the computerized spermatozoon assessment stage a spermatozoon assessment signal in response to the spermatozoon related data, wherein a sequence of digital spermatozoon images is fed into the computerized spermatozoon assessment stage as sperm related digital data and both spermatozoon motility and spermatozoon morphology are assessed by the computerized assessment stage in view of the digital spermatozoon images for generating the spermatozoon assessment signal.

In more detail, a sperm sample is diluted to an appropriate degree and transferred to the first reservoir. Then, pump 6 is controlled by the controller to pump fluid from the medium reservoir 5 into the first reservoir 2a containing the diluted sperm sample. The sperm-containing diluted fluid is thus flowing through the filter 3a towards and into the microfluidic chip 3b where it is pushed towards the observation zone. Note that the flow will be laminar, nonturbulent flow; accordingly, damaging of the spermatozoa on due to shear forces is not expected. The bolus of diluted sperm-containing fluid will then reach the observation zone and digital images may be acquired during the entire time a spermatozoon in the fluid needs to cross the observation zone.

From these images, sequences will be determined starting when a new spermatozoon enters the observation zone, that is starting with the frame where the front-most part of the sperm first appears in the field of microscopic view, the sequence lasting until the stern-most part of the tail leaves the field of microscopic view. It will be understood that where the density of sperm is so high that more than one sperm are present within the microscopic field of view on a regular basis, one or more of the same images acquired may be assigned to several sequences. It will also be understood that it is possible to identify a situation where a new spermatozoon enters the field of view, for example by detecting that at a border close to the input side into the field of view, a movement takes place and/or a new object is entering the observation zone and that in a similar manner, it can be detected that a spermatozoon leaves the field of view. As an alternative to ending an image sequence with exactly the image or frame acquired when a spermatozoon finally leaves the field of view, it would also be possible to assume that viable sperm move across the field of view within a given time, so that a fixed number of frames corresponding to this time can be set for each sequence. Alternatively, the time could be set to correspond to the time a non-motile sperm needs to pass the observation zone together with the surrounding pumped fluid.

The sequence of images is then transferred to an image processing stage where a determination is made whether or not the spermatozoon should be selected or discarded. During the time needed for making said decision and for transmitting a corresponding signal controller controlling both pumps 6 and 4, pump 6 will continue to pump medium into the first reservoir so that the bolus of liquid surrounding the spermatozoa assessed will propagate through the interconnection channel 8 to the steering zone. At the time the bolus has reached the steering zone, if the spermatozoon is to be selected, pump 6 is switched off and pump 4 is switched on, creating a flow in a direction pushing the bolus into the second outlet from the steering zone leading to the reservoir of selected spermatozoa. If the spermatozoon is judged to be unsuitable in a fertilization procedure, pump 6 is not switched off, but continues to pump and the bolus is transferred into the reservoir 2c for sperm to be discarded ("unselected sperm").

It will be understood that situations may arise where the dilution of the sperm sample is such that not only a plurality of spermatozoa are simultaneously present in the microscopic field of view but that also a plurality of spermatozoa are simultaneously present in the steering zone. As a matter of fact, given that the spermatozoa basically move within a bolus of liquid, unless the motility of the different spermatozoa simultaneously observed in microscopic field of view varies strongly, the number of spermatozoa will remain the same. As steering one spermatozoon present in the steering zone will also steer all other spermatozoa in the steering zone in the same direction, a decision can be made whether or not to steer all of the plurality of spermatozoa into the direction of selected spermatozoa if only one or a of the plurality of spermatozoa are found to have a proper morphology and motility. With respect to such decision, it should be kept in mind that according to WHO standards, the fraction of spermatozoa not usable in a fertilization procedure is about 96%, compare the literature cited above. Now, generally, even if a few spermatozoa such as a dozen spermatozoa are present in the steering zone and only 1 of those is useful for fertilization, this will still lead to a significant increase of the fraction of spermatozoa usable in a fertilization relative to the initial sample. It will also be understood that even though a small group of spermatozoa might be present in the microscopic field of view, as long as it is possible to trace the paths of the spermatozoa, the parameters for each of them could be assessed and thus a decision could be based whether to discard or select the entire group depending on the overall assessment of each member of this group.

The process of selection can be repeated and fluid containing the spermatozoa can be pumped to the observation zone using pump 6. As a matter of fact, it will be understood that pump 6 can operate in a basically continuous manner. This need not adversely affect the acquisition of images and the determination of motility provided the amount of fluid pumped in a given time remains sufficiently constant; where a risk is seen that the amount of fluid pumped by a pump 6 in a given time varies too strongly and hence the time an object such as a non-motile sperm would needed to pass through the observation zone should vary (leading to errors in the estimation of motility), the pump 6 can be stopped for the time a spermatozoon in the bolus of sperm-containing, diluted fluid needs to pass the observation zone. However, it will also be understood that motility should not only be assessed in view of the length of a path the spermatozoon will move in a given time, but that also, a specific pattern of tail movement might be required. Accordingly, the average skilled person will understand that even where the throughput of a pump varies over time, the motility could still be assessed in view of e.g. changes in the tail orientation over time.

It will also be understood that where pump 6 basically continuously pumps sperm-containing fluid through the microfluidic device but is interrupted for steering selected sperm into the reservoir for selected spermatozoa, such interruption can be taken into account when determining the motility of those spermatozoa that are upstream within the observation zone while the pump is switched off.

However, given that it is preferred to dilute the sperm-containing fluid to a degree where the density of spermatozoa is low enough to allow for observation and steering of typically single spermatozoa, it is well possible to detect the entry of a new spermatozoon to be assessed into the microscopic field of view and to switch off pump 6 in response to such detection of entry of a new spermatozoon. In this manner, optimum use is made of the dilution and the fact that pump 6 can be switched on and off at high frequencies such as 15 kHz in the embodiment shown in figure 1 without causing turbulence flow in the microfluidic device.

Once a sufficiently high number of spermatozoa have been selected and/or a sufficiently high volume of the diluted sperm sample has been assessed, in particular all of the diluted sperm sample has been assessed, the selection is ended, a corresponding signal such as an audible or visible alarm is generated and the selected spermatozoa can be used in a fertilization procedure, in particular in an ICSI fertilization procedure while at the same time, an assessment of a new sample can be initiated.

While the embodiment described above is perfectly suitable for sperm selection and gives a high throughput, variations are possible ; some of the variations will now be described.

In more detail, in the embodiment diagrammatically represented in figures 1 and 2, the microfluidic spermatozoon arrangement is laid out for a single pass of spermatozoon containing fluid; in particular, the reservoir for selected sperm need not be airtight.

In contrast to this embodiment, the embodiment of figure 3 shows that both the first reservoir for the initial sperm-containing sample and the reservoir for selected sperm each are provided with an airtight seal. Also, the econd embodiment reservoir for selected sperm differs from the corresponding reservoir of the first embodiment in that the reservoir of the second embodiment has an additional outlet connection which is however closed while selected sperm is collected therein. In this manner, and the second embodiment, once a first pass has been effected and a sufficient number of spermatozoa have been collected in the corresponding reservoir (or a sufficient volume of the initial sample has been assessed), the reservoir container for collecting selected sperm can be removed and replaced by a fresh collecting reservoir container. The removed reservoir including the sperm selected in the previous pass can then replace the first reservoir container from which sperm-containing fluid is pumped into the microfluidic chip for assessment of the sperms so that now the spermatozoa previously selected will pass the microfluidic chip a second time. In this second pass, spermatozoa will only be selected in case additional parameters also indicate that a given spermatozoa should be selected and/or if one or more parameters used in the previous selection do not just satisfy relaxed thresholds but are in conformity with more stringent limits and/or if the number of unselected sperm in the steering zone is lower than during the first pass. It will be understood that the degree to which spermatozoa selected in a previous pass shall now be rejected may depend on inter alia the amount or percentage of spermatozoa selected in the previous pass; if in the previous pass only a small number of spermatozoa could be selected and/or the overall density of spermatozoa in the initial sample and/or the quality of the spermatozoa in the initial sample was very low, the limits can be less restrictive than in a case where a particularly large number of viable spermatozoa have been identified and the second pass is only effective because too many unselected spermatozoa have been present in the steering zone together with selected sperm during the first pass.

It will be understood that in particular where a plurality of spermatozoa has simultaneously passed the observation zone and the steering zone respectively, repeating passes are particularly useful. In particular, where during the first pass, a decision has been made to simultaneously select a plurality of spermatozoa only because at least one spermatozoon was assessed to be useful for a fertilization procedure, such decision could be made in a different manner once a higher fraction of useful spermatozoa are found in the sample as is the case in a second pass. Accordingly, it is not even necessary to use different parameters during the second pass for deciding that a given single spermatozoon should be selected. Rather, it would be sufficient to only steer spermatozoa in a group of spermatozoa into the reservoir for selected spermatozoa if the fraction of selected spermatozoa in this group is sufficiently high, in particular higher than in a first pass.

Also, it would be possible to dilute the selected sperm further. This obviously allows to flush selected sperm using additional fluid into the container from which sperm to be assessed is pumped into the microfluidic chip.

An arrangement which is helpful in multi-pass selections is shown in figures 5a and 5b. Here, additional valves are provided that allow to direct pressurized fluid into the reservoir for selected sperm and to selectively allow flow from the pressurized reservoir of selected sperm back to the reservoir for sperm-containing fluid so that another pass can be effected. It will be understood that additional valves can be provided, for example a valve 4 rinsing the sperm - containing fluid container before flushing the selected sperm back into this container for a second panel.

In a further embodiment not shown, the sperms selected could immediately be subjected to another assessment in a parallel flow path after leaving the steering zone; accordingly, when handling a sample comprising a very low percentage of viable or usable sperm, it is possible to select small groups of spermatozoa wherein at least one spermatozoa is found by steering those groups to a flow path for second stage assessment while groups wherein no usable spermatozoa can be found at all are directed to a waste fluid container. The spermatozoa that are steered into the flow path for second stage assessment can then be diluted further, thus separating the spermatozoa in the group from each other, and using the diluting fluid to pump the spermatozoa of the selected groups through a second observation/selection zone along the flow path of the second stage. In this manner, it is not necessary to flush selected sperm back into the first container or rinse any of the flow paths and no additional time is needed. It will be understood that a second observation zone will require a second imaging system, but that the improved selection in the same time will easily compensate the additional costs.

While in the above description, the observation zone was separated from the steering zone by a distance sufficient to carry out an image analysis for evaluating whether or not a given spermatozoa one should be used, this need not be the case. As it has been indicated above already, it would also be possible to have a transparent steering zone and observe and evaluate spermatozoa while they are within the steering zone.

While it has been suggested above that spermatozoa are assessed in view of their morphology and their motility using a microfluidic device and steering selected spermatozoa into a corresponding reservoir, it should be noted that analyzing sequences of images in view of spermatozoon morphology and motility is also useful in an ICSI procedure where spermatozoa selected are immediately taken up with a pipette and injected into an egg. Where this needs to be done, it is possible to use a microscope, in particular a microscope autofocusing in the manner described above for acquiring a sequence of images, and to analyze the images in the manner described above assessing motility and morphology; then, rather than steering the selected sperm into a reservoir by exciting a steering pump, a live view could be generated wherein spermatozoa found to have a suitable morphology and motility are marked, for example by a green border, while spermatozoa found to be not suitable for fertilization are marked differently, for example by a red border.

It will be understood that in such a live view situation, it is vital to track the spermatozoa and that a situation may arise where spermatozoa in the field of view have not yet been assessed, for example because they are completely or partially out of focus. In such case, such spermatozoa currently unassessed could be marked in a yet third way, for example using a blue border. Note that a laboratory device implementing such method might be claimed as well.

Note in particular that even without observation zone through which the spermatozoa pass in a more or less linear manner, motility can be assessed from a sequence of images for example based on the manner the shape of the tail changes from image to image as long as a spermatozoon in one image can be identified in other images as well. It will also be understood that additional information could be considered when deciding whether or not a given spermatozoon should be (virtually) marked as selected or (virtually) marked as unselected; for example, a spermatozoon displayed on a screen could be marked green only if it is close enough to the inlet of a pipette used for injecting the spermatozoon into an egg. This prevents a laboratory technician from "hunting" spermatozoa that are too far away to be sucked into the pipette. Also, additional information could be displayed, for example using a darker shade of green for those selected spermatozoa that have a particularly high motility and particularly good morphology compared to other spermatozoa. Also, for example indications of the fraction of borderline-usable and/or very good spermatozoa could be additionally shown on a display, preferably on the same display showing the life view.

It will be understood from the above that such a method will also be a computer-assisted spermatozoon assessment method comprising the steps of feeding spermatozoon related digital data into a computerized spermatozoon assessment stage and automatically generating by the computerized spermatozoon assessment stage a spermatozoon assessment signal in response to the spermatozoon related data, wherein a sequence of digital spermatozoon images is fed into the computerized spermatozoon assessment stage as sperm related digital data and both spermatozoon motility and spermatozoon morphology are assessed by the computerized assessment stage in view of the digital spermatozoon images for generating the spermatozoon assessment signal. Also, it is to be understood that in particular for ICSI procedures, the method need only give hints to a laboratory technician or a physician while leaving the actual final decision which spermatozoon is to be used to the human being.

It will also be understood that providing a microscope with a suitable camera and image sequence assessment stage is considered inventive and that protection may be sort for such an arrangement.

Furthermore, it will be understood that while the techniques disclosed herein have been basically described with respect to the selection of human spermatozoa, for example by referring to dimensions characteristic for human spermatozoa, spermatozoa of other species such as primates or mammals might also be selected according to the principles of the present invention. In particular, this holds where the sperm quality of a species is related to both the morphology and the motility of the spermatozoa. It is noted that this is the case for a large number of animals such as apes, monkeys, bovine, horses, cattle, pigs, elephants and so forth.

## Claims

1. A computer-assisted spermatozoon assessment method
comprising the steps of
feeding spermatozoon related digital data into a computerized spermatozoon assessment stage
and
automatically generating by the computerized spermatozoon assessment stage a spermatozoon assessment signal in response to the spermatozoon related data,
wherein
a sequence of digital spermatozoon images is fed into the computerized spermatozoon assessment stage as sperm related digital data
and
both spermatozoon motility and spermatozoon morphology are assessed by the computerized assessment stage in view of the digital spermatozoon images for generating the spermatozoon assessment signal.

2. A computer-assisted spermatozoon selection method
comprising the steps of
transmitting spermatozoon related digital data for input into a computerized spermatozoon assessment stage
and
receiving a spermatozoon selection signal automatically generated by the computerized spermatozoon assessment stage in response to the spermatozoon related data, wherein
a sequence of digital spermatozoon images is transmitted for input into the computerized spermatozoon assessment stage as sperm related digital data
and
the selection signal received is indicative of an assessment of both spermatozoon motility and spermatozoon morphology in view of the digital image data.

3. A computer-assisted method, wherein a sequence of at least 3, preferably at least 4 frames are fed into the spermatozoon assessment stage.

4. A computer-assisted method according to any of the previous claims, wherein spermatozoa are sorted in response to the spermatozoon assessment signals.

5. A computer-assisted method according to the previous claim, wherein at least one fraction of spermatozoa sorted in a first assessment pass is subjected to at least one further assessment pass.

6. A computer-assisted method according to the previous claim, wherein during the or a further assessment pass, at least one of spermatozoon motility and spermatozoon morphology are assessed in a manner different from the manner of assessment during the first pass, in particular such that
during a later pass
at least one morphology characteristic different from all previously assessed morphology characteristics is assessed
and/or
a threshold for evaluation of a morphology characteristic previously assessed is set more stringent than for a previous pass
and/or
wherein a more stringent threshold for minimum spermatozoon motility is set for spermatozoa to be selected.

7. A computer-assisted method according to any of the previous claims, further comprising the step of acquiring sequences of images of spermatozoa to be assessed.

8. A computer-assisted method, wherein
a spermatozoon containing fluid is caused to flow across the field of view while the sequence of images of a spermatozoon is acquired,
and/or wherein
the field of view pictured in the images is illuminated bright enough to allow exposure times short compared to the time a spermatozoon substantially crosses the field of view pictured,
and/or wherein
the time span between two frames in a sequence of images is a fraction of the time a spermatozoon needs to cross the field of view pictured in the images.

9. A computer-assisted method wherein the fluid containing the spermatozoa to be assessed is pumped by a pump means through a microfluidic device for assessment, in particular across a field of view of an image sequence acquisition device, the pump means being intermittently operated in a manner allowing for image acquisition during those intervals while no fluid is pumped across the field of view.

10. A computer-assisted method wherein the fluid containing the spermatozoa to be assessed is pumped through a microfluidic device for assessment by a pump means, and wherein a pump means is operated in a first manner to provide a stream of fluid towards a first reservoir and is operated in a second manner to provide a stream of fluid towards a second reservoir, the first manner and the second manner being selected in response to a spermatozoon assessment signal and/or a spermatozoon selection signal.

11. A computer-assisted method according to the previous claim, wherein
the pump means comprises at least two separate pumps and
the first manner of operating the pump means comprises operating a first of the at least two separate pumps, whereas
the second manner of operating the pump means comprises operating a second pump of the at least two separate pumps
and/or wherein
the pump means comprises at least two valves,
with
a first valve of the at least two valves being opened for operating the pump means in the first manner
whereas
for operating the pump means in the second manner, a second valve of the at least two valves are opened.

12. A computer-assisted method according to any of the previous claims, wherein
a fluid containing the spermatozoa to be assessed is caused to flow thru a microfluidic device in a manner such that
during at least one of image acquisition and sorting a laminar flow environment is provided for the spermatozoa
and/or such that
the spermatozoa are maintained free of external fluid forces during at least one of image acquisition and sorting.

13. A computer-assisted method according to any of the previous claims, wherein fluid containing the spermatozoa is passed through a microfluidic device for image acquisition and/or sorting and wherein the spermatozoa in the fluid are diluted and/or singled out prior to image acquisition and/or sorting.

14. A spermatozoon sorting method based on any of the previous claims, comprising the generation of a spermatozoon assessment or selection signals for a plurality of spermatozoa and further comprising sorting spermatozoa into one of at least two different sorting reservoirs in response to the assessment signal or selection signals generated in accordance with one of the previous claims.

15. A microfluidic spermatozoa sorting device comprising
at least three fluid reservoirs for spermatozoa containing fluid,
a microfluidic flow arrangement between
a first of the three fluid reservoirs
and
the at least other two fluid reservoirs comprising
a fluid steering means for steering in a steering zone the spermatozoon containing fluid from the first reservoir to one of the at least two other fluid reservoirs,
the microfluidic flow arrangement comprising a transparent section allowing acquisition of the sequence of images of spermatozoa,
the transparent section being arranged at a distance so far away from the steering zone that that the sequence of images can be analyzed by a method according to one of the preceding claims prior to the spermatozoon reaching the steering zone,
the sorting device being adapted to apply a signal generated according to a method of one of the preceding claims to the fluid steering means for controlling the reservoir into which a spermatozoon is steered.
